# FASCICULE DE BREVET EUROPEEN

(11) **EP 2 506 724 B1**
(45) Date de publication et mention de la délivrance du brevet: **06.06.2018**
(21) Numéro de dépôt: 10795960.3
(22) Date de dépôt: 30.11.2010
(51) Int. Cl.: A61K 8/9789, A61Q 19/00, A61K 36/48, A23L 2/52, A23L 2/66, A23L 33/105, A23L 33/18, A23L 33/185, A61Q 3/02, A61Q 5/00, A61Q 7/00, A61Q 17/00, A61Q 17/04, A61Q 19/02, A61Q 19/04, A61Q 19/08

(54) **EXTRAIT DE GRAINES DE VIGNA UNGUICULATA ET COMPOSITIONS LE COMPRENANT**
VIGNA-UNGUICULATA-SAMENEXTRAKT UND ZUSAMMENSETZUNGEN DAMIT
VIGNA UNGUICULATA SEED EXTRACT AND COMPOSITIONS CONTAINING THE SAME

(30) Priorité: 30.11.2009 FR 0958529
(43) Date de publication de la demande: 10.10.2012
(73) Titulaire: Laboratoires Expanscience, 92048 Paris La Défense Cedex (FR)
(72) Inventeur: MSIKA, Philippe, F-78000 Versailles (FR); SAUNOIS, Alex, F-28210 Nogent-le-roi (FR); LECLERE-BIENFAIT, Sophie, F-28100 Dreux (FR); BAUDOIN, Caroline, F-78120 Rambouillet (FR)
(74) Mandataire: Regimbeau
(86) Numéro de dépôt international: PCT/EP2010/068573
(87) Numéro de publication internationale: WO 2011/064401

(56) Documents cités:
- EP-A1- 0 532 465
- EP-A1- 0 985 352
- EP-A2- 0 238 946
- WO-A1-02/055049
- WO-A1-2005/102259
- WO-A1-2005/105123
- WO-A1-2010/089054
- JP-A- 2002 265 324
- US-A1- 2004 131 749
- US-A1- 2009 149 362
- anonymous: "Kuhbohne, Augenbohne (Vigna unguiculata [L.] Walp. ssp. unguiculata [= V. sinensis [L.] Walp.])", , 12 juin 2007 (2007-06-12), XP002590470, Extrait de l'Internet: URL:http://geb.uni-giessen.de/geb/volltext e/2000/320/original/kuhbohne.htm [extrait le 2010-07-05]
- DATABASE Chemical Abstracts, Chemi [Online] 22 mai 1995 (1995-05-22), GOMATHINAYAGAM P ET AL: "Seed protein pattern of cowpea (Vigna unguiculata L.) and its distant species", XP002137217, extrait de CHEMICAL
- SAINI H S: "EXTRACTABILITY AND EVALUATION OF ALPHA-GALACTOSIDES OF SUCROSE IN LEGUMINOUS SEEDS", FOOD CHEMISTRY, ELSEVIER LTD, NL LNKD- DOI:10.1016/0308-8146(88)90144-6, vol. 28, no. 2, 1 janvier 1988 (1988-01-01), pages 149-157, XP000986899, ISSN: 0308-8146
- UZOGARA S G ET AL: "PROCESSING AND UTILIZATION OF COWPEAS IN DEVELOPING COUNTRIES: A REVIEW", JOURNAL OF FOOD PROCESSING AND PRESERVATION, TRUMBULL, CT, US, vol. 16, no. 2, 1 janvier 1992 (1992-01-01), pages 105-147, XP000981465,
- "Kulatthagunaah", TKDL,, 1 January 1997 (1997-01-01), XP003032285,
- "Vrddha Talakesvararasa", TKDL,, 1 January 1992 (1992-01-01), XP003032289,
- "Sitapittapathyah", TKDL,, 1 January 1996 (1996-01-01), XP003032290,
- "Bhrngarajatailam", TKDL,, 1 January 1999 (1999-01-01), XP003032291,
- "Dvitiyakulittha Gudah", TKDL,, 1 January 1996 (1996-01-01), XP003032292,
- "Medorog Cikitsa", TKDL,, 1 January 1999 (1999-01-01), XP003032293,

## Description

L'invention se rapporte à une composition comprenant un extrait de graines de *Vigna unguiculata.* L'extrait est un extrait peptidique et osidique de graines de *Vigna unguiculata.* La composition est avantageusement cosmétique, pharmaceutique, dermatologique, ou nutraceutique. L'invention a également pour objet un procédé d'extraction d'un extrait de graines de *Vigna unguiculata,* ainsi que l'extrait susceptible d'être obtenu par ledit procédé. L'invention concerne également une telle composition ou un tel extrait pour son utilisation dans la prévention ou le traitement des troubles ou pathologies de la peau, des muqueuses ou des phanères, pour son utilisation dans la prévention ou le traitement des troubles vasculaires, ou encore pour son utilisation dans la prévention ou le traitement des altérations du tissu adipeux. L'invention concerne enfin un procédé de soin cosmétique de la peau, des phanères ou des muqueuses, en vue d'améliorer leur état ou leur aspect, comprenant l'administration ou consistant à administrer une telle composition ou un tel extrait.

*Vigna unguiculata* est une légumineuse tropicale dont plusieurs sous-espèces sont très cultivées à des fins alimentaires pour leurs graines. Les cultures de cette plante qui remonteraient à 5 ou 6 000 ans font partie des plus anciennes connues pratiquées en Afrique avec le sorgho et le millet. Signalons dès à présent des confusions probables figurant dans des documents et ouvrages entre noms vernaculaires de sous espèces.

### La plante Vigna unguiculata

De nom botanique *Vigna unguiculata* (L.) Walp., *Vigna unguiculata,* appartenant à l'ordre des Fabales et à la famille botanique des Fabacées, présente des organes tous bien caractéristiques des légumineuses. Cependant, il existe des différences morphologiques d'un cultivar à l'autre. Les noms usuels de plante sont Niébé, pois à vache, cornille, dolique à oeil noir, haricot indigène et le terme anglais « cowpea » désigne l'espèce *V. unguiculata* ainsi que toutes ses sous-espèces.

*V. unguiculata* est une plante herbacée allongée, dressée ou bien grimpante, mesurant en moyenne 30 à 60 cm (extrêmes : 15-80 cm) de haut. Les feuilles sont alternes, trifoliées, avec un pétiole de 5 à 25 cm de long. Les folioles latérales sont opposées et asymétriques tandis que la foliole centrale est symétrique, ovale, et souvent plus grande. Leurs tailles et formes varient. Elles sont lisses, mates ou brillantes, rarement pubescentes.

L'inflorescence est rameuse avec de longs pédoncules terminés par des fleurs de couleur blanche, crème, jaune, mauve ou pourpre.

Les fleurs, classiques de légumineuses, contiennent un nectar qui contribue à l'attirance des insectes. Les gousses, réunies par 2 ou 3, voire davantage sur chaque pédoncule floral, sont dures et fermes, cylindriques, resserrées entre les graines, droites à légèrement arquées, et mesurent 10 à 25 cm de long. Elles contiennent 10 à 15 graines. Lorsque les graines deviennent matures, la couleur des gousses peut différer : communément vertes, jaunes ou pourpres. Lorsque les graines sont sèches, les gousses deviennent brun-roux ou brunes.

*V. unguiculata* est cultivée dans les régions équatoriales, tropicales et subtropicales situées entre les parallèles 35° Nord et 30° Sud. Il s'agit d'une culture de saison chaude bien adaptée à de nombreuses régions des tropiques humides. Elle est peu exigeante au plan du sol.

### Caractéristiques de la graine

Les graines ont des tailles et des formes variables plus ou moins oblongues liées à la compression exercée par la gousse : en forme de rein en cas de gousses lâche, plus globulaire en cas de compression. Leurs couleurs sont très variées : blanche, ivoire, verte, chamois, rouge, brune ou noire. Elles peuvent être mouchetées ou tachetées par une couleur différente et présentent la particularité d'une tache noire autour du hile (d'où certaines appellations vernaculaires : ex. Dolique à oeil noir).
La cuticule de la graine peut être lisse ou ridée. La taille des graines varie de 6 à 10 mm de long sur 4 à 7 mm de large. 100 graines pèsent de 11 à 26 grammes (Henshaw, 2008).

Les teneurs en protéines figurant dans la littérature consultée sont élevées. Elles se situent entre 19,5 et 27,3 % m/m. Au niveau des acides aminés, les graines de *V. unguiculata* étaient considérées en 1959 comme assez bien équilibrées en acides aminés essentiels (Busson et al., 1959). Cependant, selon les données plus récentes et c'est le cas de toutes les légumineuses, elles apparaissent déficitaires en acides aminés soufrés (méthionine et cystine) comparées aux protéines animales et selon les recommandations de la FAO/WHO (Olivera-Castillo et al., 2007 ; Maia et al., 2000 ; Hussain MA, Basahy, 1998). Ces deux acides aminés essentiels et soufrés apparaissent pour cette graine comme les premiers facteurs limitants chez les enfants de 2 à 5 ans (Maia et al., 2000).

En 1980, une globuline majoritaire, hétérogène, d'un poids moléculaire de 170 000 est signalée (Khan et al., 1980). Les globulines constituent plus de 51 % des protéines de la graine et les albumines approximativement 45%. Par électrophorèse ou chromatographie anionique, les globulines se répartissent en 3 composants principaux, α-,β et γ-vignine (Freitas et al., 2004). Freitas et al., 2004 donnent d'autres précisions au sujet des propriétés de ces protéines (caractéristiques chimiques, coefficient de sédimentation, analyse immunologique, hémagglutination).
Les teneurs en glucides des graines de niébé ont été bien moins publiées que celles des protéines car beaucoup plus courantes pour des graines végétales.
Sucres simples (monomères ou dimères) :
- Fructose, glucose, mannitol, inositol (Hussain et Basahy, 1998).
- Saccharose (0,7-4,6 %) sur 10 échantillons du Nigeria (Nwinuka et al., 1997).
Oligosaccharides :
- Stachyose (2,4-4,1 %) et Raffinose (1,2-6,8 %) sur 10 échantillons. du Nigeria (Nwinuka et al., 1997 ; Onigbinde et Akinyele, 1983).
- Verbascose (Hussain et Basahy, 1998).

Les teneurs en lipides des graines sont faibles, ne dépassant pas 3,5 %.

Autres constituants des graines :
- Fibres alimentaires : 1,7-4,5 % (6 variétés du Nigeria - Onwuliri et Obu, 2002) 2,6 % (échantillon du Mexique - Rivas-Vega et al., 2006) 15,8 %; dont 13,1% insolubles 2,7% solubles (Kahlon et Shao, 2004)
- Tanins : 0-0,2 % (6 variétés du Nigeria - Onwuliri et Obu, 2002) 0,12-0,14 % (variétés du Nigeria - Ene-Obong, 1995)
- Oxalate : 0,8-1,71 % (6 variétés du Nigeria - Onwuliri et Obu, 2002)
- Phytates : 0,24-1,41 % (6 variétés du Nigeria - Onwuliri et Obu, 2002) 0,84-0,99 % (variétés du Nigeria - Ene-Obong, 1995)
- Minéraux : 3,3-4,2 % (Henshaw, 2008)
   Teneurs courantes des différents minéraux. Des teneurs notoires en fer allant de 5,6 à 15 mg/100 g (Singh et al., 2002) et 5 à 15 mg/100 g (Oluwatosin, 1998) dans les graines sèches sont citées; mais pas par tous : 1,6 mg/100 g pour un échantillon Turque (Kabas et al., 2007).
- Uréides :
   Selon une étude, l'allantoïne et l'acide allantoïque représentent les fractions principales du pool azoté soluble des nodules sur racines de *Vigna unguiculata* [L.] Walp. cv. Caloona) pendant la croissance végétative et la reproduction. La tige et les pétioles sont apparus comme les principaux emplacements de l'accumulation de ces uréides, particulièrement en début de fructification (Teneurs non explicites). Dans d'autres légumineuses, les uréides prédomineraient dans les graines (ex. *Phaseolus mungo* L., *Dolichos spp*., *Glycine max* [L.] M).

Des analyses nutritionnelles, réalisées par la Demanderesse, sur des graines de différentes origines montrent qu'elles peuvent contenir par exemple :
- 22% de fibres
- 22% de protéines
- 1% de lipides
- 3,4% de saccharose

### ART ANTERIEUR

### Usages traditionnels

L'utilisation à des fins alimentaires constitue l'emploi traditionnel majeur des graines de niébé dans le monde.

### Alimentation humaine (Singh et al., 2002)

La graine de *V. unguiculata* est un aliment nutritionnel important pour une partie des populations vivant en climat tropical. A titre d'exemple, chaque nigérian mange du niébé et la consommation par habitant est d'environ 25 à 30 kg par an. Au Brésil, la consommation serait de 20 kg / habitant / an.

Aux USA, après nettoyage et conditionnement, les graines de cowpeas et purpleeye sont couramment vendues au public en tant qu'aliments. Dans ce pays, on les trouve également sous forme de conserves, de soupes ou bien mélangées à d'autres légumes.

En Afrique, la graine sèche est communément moulue et consommée dans plusieurs plats traditionnels Africains, comme la bouillie, le pain, l'aliment de sevrage pour enfants ou encore transformée en beignets. Cette utilisation traditionnelle est parfaitement décrite dans des rapports de M. Ouétian Bognounou, Officier et Chevalier de l'Ordre National, Chevalier des Palmes académiques, chargé de recherche en floristique et ethnobotanique de l'INERA du Burkina Faso co-écrits avec M. Marc Olivier, consultant en ethnobotanique.

A noter que les jeunes feuilles et les gousses immatures sont aussi utilisées en guise de légumes dans des pays tropicaux.

### Alimentation animale (Singh et al., 2002)

Dans beaucoup de régions tropicales, la plante par elle-même est le seul fourrage de haute qualité disponible pour nourrir le bétail. Les parties vertes de la plante comme les fanes après la récolte des graines, peuvent être utilisées pour l'alimentation des lapins.

Elles sont sources de protéines (17-30% / MS), de fibres (18-29% de cellulose brute / MS). Les gousses vides obtenues après battage des grains forment aussi une source intéressante de fibres (32-35% de cellulose brute / MS), mais sont plus pauvres en protéines (12-14% / MS). (Djago et al., 2007).

### Usages en médecine traditionnelle

En Chine, les graines ne semblent pas être utilisées comme drogue médicinale contrairement aux gousses sans graine et à la plante (Duke et Ayensu, 1985).
En Afrique, et plus particulièrement au Burkina Faso, les feuilles peuvent être utilisées en cataplasmes contre certaines dermatoses et enflures.

EP 0 238 946 divulgue un isolat de protéines, contenant de préférence au moins 83% en poids de protéines, ayant un poids moléculaire supérieur à 20 000 Dalton. Il ne s'agit ainsi pas de peptides extraits de Vigna, mais de protéines. Dans le procédé selon EP 238 946, des enzymes protéolytiques sont utilisées pour favoriser la coagulation des protéines dans l'isolat de protéines en favorisant le clivage des liaisons peptidiques. Les enzymes protéolytiques utilisées sont notamment choisies parmi la présure standard, la présure microbienne, la rénine, la chymosine, la pepsine et la papaïne.

EP 0 532 465 décrit des fractions protéiques de graines de légumineuses. La plante Vigna est citée, mais pas l'espèce spécifique *Vigna unguiculata.*

WO 2005/105123 concerne un médicament qui comprend un extrait peptidique d'avocat et qui peut également comprendre une composition contenant de la D-mannoheptulose et / ou du perséitol.

En outre, les documents XP003032289 (Vrddha Talakesvararasa), XP003032291 (Bhrngarajatailam) et XP003032292 (Dvitiyakulittha Gudah) portent sur des décoctions à partir de *Vigna Unguiculata.*

### DESCRIPTION DE L'INVENTION

La Demanderesse a découvert que des extraits de graines de *Vigna unguiculata,* tels que des extraits peptidiques et osidiques, présentent des propriétés cosmétiques et dermatologiques jamais décrites jusqu'à présent. En particulier, c'est la première fois que des extraits peptidiques et osidiques de *Vigna unguiculata* sont utilisés en tant que tels, pour leurs propriétés spécifiques.

L'invention a pour objet une composition comprenant un extrait de graines *de Vigna unguiculata,* ledit extrait de graines comprenant un mélange de peptides et de sucres, tel qu'un extrait peptidique et osidique de graines *de Vigna unguiculata,* éventuellement en association avec un excipient approprié.

La composition est avantageusement cosmétique, pharmaceutique, dermatologique ou nutraceutique. Ladite composition est de préférence formulée pour être administrée par voie topique externe ou orale.

L'extrait de graines de *Vigna unguiculata* selon l'invention est un extrait peptidique et osidique.

Par « extrait peptidique et osidique », on entend un extrait comprenant majoritairement ou essentiellement des peptides et des oses (sucres).

Avantageusement, l'extrait de graines de *Vigna unguiculata* est constitué essentiellement d'un mélange de peptides et de sucres.

L'extrait de graines de *Vigna unguiculata* selon l'invention est substantiellement débarrassé de toute protéine native résiduelle, car ces protéines peuvent provoquer des réactions allergiques que l'on souhaite éviter.

Typiquement, l'extrait de graines de *Vigna unguiculata* est substantiellement débarrassé des acides aminés libres.

L'extrait selon la présente invention comprend 10 à 90 % en poids de peptides et 10 à 90 % en poids de sucres totaux, les pourcentages étant exprimés par rapport au poids total dudit extrait.

Dans un mode de réalisation particulier de la présente invention, l'extrait comprend 10 à 50 %, avantageusement 20 à 45 %, typiquement 20 à 40 %, en particulier 30 à 40 % en poids de peptides.

Dans un autre mode de réalisation particulier de la présente invention, l'extrait comprend 20 à 80 %, avantageusement 30 à 70 %, typiquement 50 à 60 %, en poids de sucres.

Selon un aspect préféré de l'invention, l'extrait peptidique et osidique comprend 30% à 40 %, par exemple 30 % en poids de peptides et 50 à 60%, par exemple 60 %, en poids de sucres. Les pourcentages sont exprimés typiquement par rapport au poids total de matière active dudit extrait avant ajout par exemple d'un éventuel support de séchage.

Selon une caractéristique particulière de la présente invention, le ratio peptides/sucres de l'extrait est inférieur ou égal à 1, et avantageusement compris entre 0,5 et 1.

Selon une variante avantageuse de l'invention, la composition contient 0,001 à 10 %, typiquement 0,01 à 5 %, en poids d'extrait, exprimé en pourcentage d'extrait sec.

L'invention a également pour objet un procédé de préparation d'un extrait peptidique et osidique de graines de *Vigna unguiculata.*

Avantageusement selon l'invention, le procédé de préparation d'un extrait peptidique et osidique de graines de *Vigna unguiculata* comprend les étapes successives suivantes :
- broyage des graines de *Vigna unguiculata,*
- dispersion des graines de *Vigna unguiculata* broyées dans de l'eau ou en phase aqueuse, avantageusement à un pH compris entre 3.0 et 9.0 et à une température comprise entre 20 et 90°C,
- hydrolyse enzymatique de ladite dispersion, et
- récupération de l'extrait peptidique et osidique.

Suite à la dispersion en phase aqueuse, on réalise une hydrolyse enzymatique.

L'hydrolyse enzymatique est réalisée par une ou des enzymes adaptées dans les conditions optimales de pH et de température, connues de l'homme de l'art, par exemple à un pH compris entre 3.0 et 9.0 et typiquement à une température comprise entre 20 et 90°C, par un mélange de protéases et de carbohydrases, telles que des pectinases, cellulases, arabanases, hémicellulases, xylanases et β-glucanases, puis on récupère l'extrait peptidique et osidique.

L'hydrolyse enzymatique de la dispersion peut être suivie si besoin d'un traitement thermique afin de dénaturer les enzymes, typiquement entre 80 et 100°C.

L'étape d'hydrolyse du procédé selon l'invention est très importante, puisqu'elle permet de transformer ou de « découper » les protéines natives présentes dans les graines de Vigna en peptides. Cette étape permet également avantageusement de transformer ou de « découper » les polysaccharides présents dans les graines de Vigna en oligosaccharides ou monosaccahrides.

De manière particulièrement avantageuse selon l'invention, le procédé comprend une étape d'hydrolyse enzymatique, puis une étape d'ultrafiltration par exemple à un seuil de coupure compris entre 10000 Daltons et 15000 Daltons, pour éliminer les protéines résiduelles qui sont potentiellement allergisantes et éventuellement les enzymes.

Dans un mode de réalisation particulier selon l'invention, le procédé comprend également une étape de nanofiltration avec par exemple un seuil de coupure compris entre 100 Daltons et 300 Daltons, avantageusement entre 130 et 300 Daltons, typiquement entre 200 Daltons et 300 Daltons, pour éliminer les acides aminés libres ou les sels minéraux, suite à l'étape d'ultrafiltration.

Selon une variante avantageuse de l'invention, suite à l'hydrolyse de la dispersion et préalablement à la récupération de l'extrait peptidique et osidique, on procède à une filtration ou à une centrifugation, éventuellement suivie d'une ultrafiltration, diafiltration, et/ou nanofiltration.

Préférentiellement, à titre d'exemple, l'extrait peptidique et osidique peut être obtenu selon le procédé suivant :
a) mise en solution des graines broyées à 10% de matière sèche dans l'eau ;
b) hydrolyse enzymatique des carbohydrates par action combinée d'une amylase (Amylyve AN30 de la société Lyven par exemple) et d'un autre mélange de carbohydrases à activités complémentaires, telles que arabanase, β glucanase, hémicellulase, xylanase (Viscozyme L de la société Novozymes par exemple) dans les conditions de pH et température optimales pour l'activité de ces enzymes ;
c) suivi d'une hydrolyse par une protéase alcaline (par exemple la Prolyve 1000 de la société Lyven) ;
d) traitement thermique afin de dénaturer les enzymes ;
e) centrifugation, ultrafiltration et/ou diafiltration sur membranes 15 kDa afin d'éliminer les protéines résiduelles potentiellement allergisante;
f) nanofiltration sur membrane 200 Da afin d'éliminer des sels minéraux ou des acides aminés libres par exemple.

Avantageusement selon l'invention, suite à la récupération de l'extrait peptidique et osidique, on procède à au moins l'une des étapes suivantes :
- décoloration de l'extrait ainsi obtenu, par exemple en présence de charbon actif ou par tout autre moyen connu de l'homme de l'art, et
- séchage de l'extrait obtenu sur un support ou sans support.

Avantageusement, l'extrait peptidique et osidique peut être séché selon des procédés connus de l'homme de l'art en présence ou non d'un support de type, par exemple, maltodextrines ou fibres d'acacia (Fibregum® société CNI) ; typiquement selon un ratio pouvant varier 0% à 80% de support par rapport au pourcentage de matière sèche obtenu dans la forme liquide de l'extrait et préférentiellement séché par lyophilisation afin d'obtenir dans la poudre finale, 50% de matière sèche issue de l'extrait et 50% de support de lyophilisation.

### Exemple d'extrait peptidique et osidique liquide ainsi obtenu:

### 1 - Analyse physico-chimique (% / matière sèche totale)

| | | |
|---|---|---|
| Extrait sec (2h, 105°C, étuve ventilée) | : | 8.9% |
| pH | : | 4.0 |
| Azote α-aminé (OPA, équivalent leucine) | : | 13% |
| Peptides (Kjeldahl, N x 6.25) | : | 28% |
| Sucres solubles (HPLC) | : | 60% |
| Cendres totales | : | 7% |

### 2 - Profil des répartitions des masses molaires des peptides solubles

| | | |
|---|---|---|
| Inférieur à 130 Da | : | 24% |
| Entre 130 - 300 Da | : | 14% |
| Entre 300 - 1200 Da | : | 48% |
| Entre 1200 - 3500 Da | : | 13% |
| Supérieur à 3500 Da | : | ≤1% |

### 3 - Dosage d'oligosaccharides spécifiques (% / matière sèche totale)

Raffinose : 5.9%
Stacchyose : 2.4%
Verbascose : 3.15%

La présente invention a également pour objet un extrait de graines de *Vigna unguiculata* susceptible d'être obtenu par le procédé mentionné ci-dessus. Un tel extrait contient 10 à 90 % en poids de peptides et 10 à 90 % en poids de sucres.

Dans un mode de réalisation particulier de la présente invention, l'extrait comprend 10 à 50 %, avantageusement 20 à 45%, typiquement 20 à 40 %, en particulier 30 à 40 %, en poids de peptides.

Dans un autre mode de réalisation particulier de la présente invention, l'extrait comprend 20 à 80 %, avantageusement 30 à 70 %, typiquement 50 à 60%, en poids de sucres.

Selon un aspect préféré de l'invention, l'extrait peptidique et osidique comprend 30% à 40 %, par exemple 30%, en poids de peptides et 50 à 60%, par exemple 60%, en poids de sucres.

Selon une caractéristique particulière de la présente invention, le ratio peptides/sucres de l'extrait est inférieur ou égal à 1, et avantageusement compris entre 0,5 et 1.

Typiquement, les sucres de l'extrait sont des monosaccharides ou oligosaccharides, tels que du raffinose, du stacchyose, et du verbascose, ou leurs mélanges. En particulier, les sucres de l'extrait sont composés majoritairement d'un mélange de raffinose, de stacchyose, et de verbascose.

Avantageusement selon l'invention, les peptides de l'extrait présentent une masse moléculaire inférieure ou égale à 3500 Dalton. En particulier, les peptides de l'extrait présentent majoritairement une masse moléculaire inférieure ou égale à 1200 Dalton, typiquement entre 300 et 1200 Dalton.

L'extrait peptidique et osidique selon l'invention ne contient substantiellement pas de protéines résiduelles qui sont potentiellement allergisantes.

Dans un mode de réalisation particulier, l'extrait peptidique et osidique selon l'invention ne contient substantiellement pas d'acides aminés libres.

Selon un autre aspect de l'invention, la composition peut en outre comprendre au moins un autre composé actif en plus de l'extrait de graines de *Vigna unguiculata.*

Cet autre composé peut être choisi parmi tous les composés et leurs équivalents fonctionnels, énoncés ci-dessous.

Cet autre composé peut être en particulier choisi parmi des actifs classiquement utilisés en dermatologie ou cosmétique tels que les émollients, les actifs hydratants, les kératorégulateurs, les kératolytiques, les agents restructurant de la barrière cutanée, des agonistes PPARs (ou Peroxysome Pro liferator Activated Receptor), les agonistes RXR ou LXR, les agonistes des récepteurs à la vitamine D ou aux corticoïdes, les activateurs de la différenciation des kératinocytes (rétinoïdes, calcidone®, le calcium), les agents sébo-régulateurs, les agents anti-irritants, les agents apaisants, les agents anti-inflammatoires, les agents anti-oxydants et les agents anti-âge.

Cet autre composé peut aussi être choisi parmi des principes actifs ayant une action thérapeutique ou cosmétique complémentaire, tels que les antibiotiques, les pré et probiotiques, les agents anti-bactériens, les composés antifongiques, les agents anti-viraux, les conservateurs, les immunomodulateurs (tacrolimus ou pimécrolimus), les oxazolines, les facteurs de croissance, les agents cicatrisants ou les molécules eutrophiques, les médicaments, les agents pigmentants ou hypopigmentants, les agents lipolytiques ou inhibiteurs de la lipogénèse ou encore les agents anti-cellulite ou amincissants, les filtres et écrans solaires minéraux ou organiques (pigmentaires ou ultrafins), des aliments classiques ou fonctionnels : hyper ou hypoglycémiants, des nutriments anti-graisse ou anti-cellullite, anti-cholestérol, anti-oxydant, énergisant, reconstituant, ou ayant un impact sur les signes secondaires de la ménopause.

Cet autre composé peut aussi être choisi parmi des extraits naturels de plante (parties de végétaux extractibles en phase aqueuse ou huileuse : polyphénols, flavonoides, autres peptides et sucres, .....), des composés contenant des insaponifiables d'huiles végétales, des insaponifiables stéroliques ou des produits pouvant en contenir (insaponifiables d'huiles végétales, notamment insaponifiables d'huile de Soja, insaponifiables de beurres végétaux ou de matières butyreuses et leurs mélanges, insaponifiables de cires naturelles, insaponifiables d'extraits huileux, insaponifiables de co-produits huileux industriels, insaponifiables d'extraits de corps gras d'origine animale, insaponifiables d'huiles marines, insaponifiables d'extraits de la matière grasse lactique, insaponifiables de lipides extraits d'organismes unicellulaires, insaponifiables des lipides extraits des algues et organismes marins, etc), des stérols, des stanols, des phytostérols, des phytostanols, des tocophérols, des concentrats d'huiles de Tournesol, de Colza et/ou de Palme, des oligo-éléments, des vitamines, des acides gras en oméga 3, 6 ou 9, des plantes hypoglycémiantes ou hyperglycémiantes ou sucrantes.

Les actifs hydratants / émollients les plus utilisés sont la glycérine ou ses dérivés, l'urée, l'acide pyrrolidone carboxylique et ses dérivés, l'acide hyaluronique de tout poids moléculaire, les glycosaminoglycanes et tout autres polysaccharides d'origine marine, végétale ou biotechnologique comme par exemple la gomme xanthane, le fucogel®, certains acides gras comme l'acide laurique, l'acide myristique, les acides gras poly- et mono-insaturés de type oméga 3, 6 et 7, 9 comme l'acide linoléique et acide palmitoléique, l'oléodistillat de Tournesol, les peptides d'Avocat, le beurre de Cupuaçu. Les modulateurs de la différenciation épidermique, protéines clés du *stratum corneum* ou *granulosum*, pouvant être utilisés en association sont avantageusement les rétinoïdes, les peptides de Lupin, les sucres d'Avocat, ou un extrait peptidique de Quinoa.
Les agents anti-inflammatoires/anti-irritants, apaisants les plus classiques sont l'acide glycyrrhétinique (les dérivés de réglisse) avec ses sels et esters, l'acide lipoïque, le beta-carotène, la vitamine B3 (niacinamide, nicotinamide), la vitamine E, la vitamine C, la vitamine B12, les flavonoïdes (thé vert, quercétine...), le lycopène ou la lutéine, les sucres d'Avocat, l'oléodistillat d'Avocat, l'arabinogalactane, les peptides de Lupin, un extrait total de Lupin, un extrait peptidique de Quinoa, le Cyclocéramide® (dérivé d'oxazoline), les isoflavones comme par exemple la génistéine/génistine, daidzéine/daidzine, les eaux de source ou thermales (eau d'Avène, eau de la Roche Posay, eau de Saint Gervais, eau d'Uriage, eau de Gamarde), les extraits de Goji (*Lycium barbarum*), les peptides ou complexes d'acides aminés végétaux ou encore la disulone topique, ou les médicaments anti-inflammatoires stéroïdiens (AIS), tels que les corticoïdes, ou non-stéroïdiens (AINS).
Parmi les agents kératorégulateurs/kératolytiques les plus utilisés se trouvent : les acides de fruits alpha hydroxyacide - AHA (acide citrique, acide glycolique, acide malique, acide lactique...), les esters d'AHA, les associations d'AHA avec d'autres molécules comme l'association acide malique et protéines d'amandes (keratolite®), l'association acide glycolique ou acide lactique avec l'arginine ou encore l'association d'hydoxy-acide avec des molécules lipidiques comme le LHA® pour lipo-hydroxy-acide, les complexes d'hydroxyacide amphotères - AHCare, l'acide azélaïque et ses sels et esters, l'écorce de saule (*Salix alba* bark extract), l'acide salicylique (beta hydroxyacide - BHA), et ses dérivés comme l'acide capryloyl salicylique ou en association avec d'autres molécules comme l'association acide salicylique et polysaccharide le tazarotène, l'adapalène, ainsi que les molécules de la famille des rétinoïdes tels que : la trétinoïne, le rétinaldéhyde, l'isotrétinoïne, ou le rétinol.
Les agents sébo-régulateurs pouvant être utilisés en association sont avantageusement choisis dans le groupe constitué par les inhibiteurs de 5-alpha réductase comme par exemple l'actif 5-alpha Avocuta®. Le zinc (et ses sels gluconate, salicylate et acide pyroglutamique) présente aussi une activité sébo-suppresseur. On peut citer aussi la spironolactone, anti-androgène et antagoniste de l'aldostérone, qui engendre une réduction significative du taux de sécrétion de sébum. D'autres molécules telles que par exemple *Cucurbita pepo*, extraite des graines de citrouille, et l'huile de pépins de courge, le sabal, limitent la production de sébum par inhibition de la 5α-réductase. D'autres séborégulateurs d'origine lipidique agissant sur la qualité du sébum, comme l'acide linoléique présentent un intérêt.

Les facteurs de croissance pouvant être utilisés en association sont avantageusement la becaplermine et le TGF-beta, l'EGF, le NGF, le VEGF.
On entend par agent antioxydant une molécule qui diminue ou empêche l'oxydation d'autres substances chimiques. Les antioxydants pouvant être utilisés en association sont avantageusement choisis dans le groupe constitué par les thiols et les phénols, par les dérivés de réglisse comme l'acide glycyrrhétinique avec ses sels et esters, l'alpha bisabolol, l'extrait de ginkgo biloba, de calendula, le Cyclocéramide® (dérivé d'oxazoline), les peptides d'Avocat, les oligo-éléments comme le cuivre, le zinc, et le sélénium, l'acide lipoïque, la vitamine B12, la vitamine B3 (niacinamide, nicotinamide), les vitamines C, les vitamines E, le co-enzyme Q10, le krill, le glutathion, le BHT pour butylhydroxytoluène, le BHA pour butylhydroxyanisol, le lycopène ou la lutéine, le beta-carotène, la grande famille des polyphénols comme les tanins, les acides phénoliques, les anthocyanes, les flavonoïdes avec par exemple les extraits de thé vert, de fruits rouges, de cacao, de raisin, de *Passiflora incarnats*, de *Citrus* ou encore les isoflavones comme par exemple la génistéine/génistine, la daidzéine/daidzine. Dans le groupe des anti-oxydants, on trouve aussi les substances anti-glycation telles que la carnosine ou certains peptides, la n-acétyl-cystéine, ainsi que les enzymes antioxydants ou antiradicalaires comme la SOD (super oxyde dismutase), la catalase, la glutathion peroxydase, la thioredoxine reductase et leurs agonistes.
Les agents cicatrisants et restructurants de la barrière cutanée, permettant de stimuler la synthèse des lipides clés de l'épiderme, et pouvant être utilisés en association sont avantageusement la vitamine A, le panthénol (vitamine B5), les sucres d'Avocat, le Lupéol, l'extrait peptidique de Maca, un extrait peptidique de Quinoa, l'arabinogalactane, l'oxyde de zinc, le magnésium, le silicium, l'acide madécassique ou asiatique, le sulfate de dextran, le coenzyme Q10, la glucosamine et ses dérivés, la chondroïtine sulfate et globalement les glucosaminoglycanes ou GAG, le sulfate de dextran, les céramides, le cholestérol, le squalane, les phospholipides, les peptides de soja fermenté ou non, les peptides végétaux, les polysaccharides marins, végétaux ou biotechnologiques comme les extraits d'algues ou l'extrait de fougère, les oligo-éléments, les extraits de plantes à tanin comme les tanins dérivant de l'acide gallique appelés tanins galliques ou encore hydrolysables, initialement trouvés dans la noix de galle, et les tanins catéchiques résultant de la polymérisation d'unités flavaniques dont le modèle est fourni par le Cachou (*Acacia catechu*). Les oligo-éléments pouvant être utilisés sont avantageusement choisis dans le groupe constitué par le cuivre, le magnésium, le manganèse, le chrome, le sélénium, le silicium, le zinc et leurs mélanges. Peuvent également être utilisés des concentrats de Tournesol, plus avantageusement des concentrats de Tournesol linoléiques, tels que l'actif commercialisé par les Laboratoires Expanscience, Soline®, des insaponifiables d'huile végétale, tel que l'Avocadofurane®, ou des agonistes PPARs (rosiglitazone, pioglitazone), RXR, LXR.
Les agents anti-âge pouvant agir sur les sujets d'âge mûr sont des agents antioxydants et en particulier la vitamine C, ou encore la vitamine A, le rétinol, le rétinal, l'acide hyaluronique de tout poids moléculaire, l'Avocadofurane®, les peptides de Lupin, l'extrait peptidique de Maca.
Les composés antifongiques pouvant être utilisés en association sont avantageusement l'econazole et le ketoconazole.
Les conservateurs antiseptiques pouvant être utilisés en association sont par exemple le triclosan, la chlorhéxidine, les ammoniums quaternaires.
Les antibiotiques pouvant être utilisés en association sont avantageusement l'acide fucidique, la pénicilline, les tétracyclines, la pristinamycine, l'érythromycine, la clindamycine, la mupirocine, la minocycline, la doxycycline. Les agents anti-viraux pouvant être utilisés en association sont avantageusement l'acyclovir et le valacyclovir. Les conservateurs pouvant être utilisés en association sont par exemple ceux généralement utilisés en cosmétique ou en nutraceutique, les molécules à activité anti-bactérienne (pseudo-conservateurs) tels que les dérivés capryliques comme par exemple le capryloyl glycine et le glycéryl caprylate, tels que l'hexanediol, et le sodium levulinate, les dérivés de zinc et de cuivre (gluconate et PCA), la phytosphingosine et ses dérivés, le peroxyde de benzoyle, la piroctone olamine, le pyrithione zinc, le sulfure de sélénium.
Les actifs protecteurs solaires pouvant être utilisés en association sont avantageusement des filtres ou écrans solaires UVB et/ou UVA ; tels les écrans ou filtres minéraux et/ou organiques connus de l'homme du métier qui adaptera leur choix et leurs concentrations en fonction du degré de protection recherché.
A titre d'exemple d'actifs protecteur solaire, on peut notamment citer le dioxyde de titane, l'oxyde de zinc, le méthylène bis-benzotriazolyl tétraméthylbutylphénol (nom commercial : TINOSORB M) et le Bis-éthylhéxyloxyphénol méthoxyphényl triazine (nom commercial : TINOSORB S), l'octocrylène, le butyl méthoxydibenzoylméthane, l'acide terephthalylidene dicamphor sulfonique, le 4-méthylbenzylidène de camphre, le benzophénone, l'éthylhexyl méthoxycinnamate, l'éthylhexyl diméthyl PABA, le diéthylhexyl butamido triazone.
Les agents amincissants pouvant être utilisés en association sont avantageusement la caféine, le fucus, les extraits végétaux comme par exemple : l'extrait de lierre, de cacao, de guarana, de petit houx, de thé vert, de maté, de poivre de sichuan, de marron d'inde, la *Centella asiatica*, la carnithine, la glauscine, l'escine, l'extrait de petit houx (*Ruscus esculentus*), les isoflavones comme par exemple la génistéine, le Ginko biloba, la forskoline, le rétinol et autres rétinoïdes, la phloridzine, la criste marine peuvent également être utilisés en association.
Les agents anti-chute des cheveux et/ou fortifiants pour les cheveux et les ongles sont avantageusement les phytostérols, les isoflavones comme par exemple les isoflavones de soja, le RTH16®, l'aminexil®, le minoxidil®, le rétinol, le zinc et ses dérivés, la neoruscine, la vitamine E, la vitamine B2, la vitamine B3, la vitamine B6, la vitamine PP, la vitamine B5 (panthénol, bépanthène), la vitamine B8 (vitamine H ou biotine), la vitamine B9 (acide folique), l'alpha hydroxyacide, la quinine, certains acides aminés comme la cystéine, la cystine, la méthionine. Les inhibiteurs de 5-alpha réductase tels que par exemple le finastéride, le dutastéride, *Serenoa serrulata* ou *repeins*, l'extrait de *Cucurbita pepo* ou encore /certains phytostérols, peuvent également être utilisés en association. La kératine, les oligoéléments, ou les sels minéraux peuvent également être utilisés en association. Certains extraits protéiques ou lipidiques végétaux comme par exemple les extraits de pfaffia, de sauge, de citron, de ginseng, de quinquina, de jojoba, de marron d'inde, de miel, de blé, d'ortie, d'échinea, de noix de coco peuvent également être utilisés en association.
Les agents anti-pelliculaires (cuir chevelu) sont avantageusement l'extrait de capucine, la vitamine F, le thymol, l'argile, le pyrithione de zinc, le zinc-PCA, le gluconate de zinc, le sulfate de zinc, le camphre, l'extrait de myrte, l'acide salicylique, la vitamine B5, le climbazole, l'ichtyol, le sélénium et ses dérivés, l'extrait de Curbicia, l'extrait de Carthame, l'extrait d'huile de Melaleuca, l'huile de Bourrache et de *Mimosa Tenuiflora*,

la propolis, Kertyol, l'acide glycolique, le kéluamid, la cyclopiroxolamine, le piroctone olamine, le capryloyl glycine, le 5 alpha Avocuta.
Les médicaments ou agents cosmétiques pouvant être utilisés en association sont avantageusement les médicaments ou les agents cosmétiques appropriés pour une administration par voie topique ou orale, en particulier pour la prévention et/ou le traitement de l'atopie/eczéma (les corticostéroïdes comme l'hydrocortisone, le desonide, l'acetonide de fluocinolone, le propionate de fluticasone, les immunomodulateurs topiques inhibiteurs de la calcineurine comme le tacrolimus et le pimecrolimus, la cyclosporine, l'azathioprine, le méthotrexate, la vitamine B12, les molécules anti-microbiennes, les anti-histaminiques comme l'hydroxyzine et la diphenhydramine, les antibiotiques, les pré- et pro-biotiques, la naltrexone, les agonistes de PPAR alpha tels que l'oléodistillat de Tournesol, les émollients contenant des céramides ou autres lipides clés épidermiques), de l'acné (les antibiotiques, le péroxyde de benzoyle, les rétinoïdes, l'acide azélaïque, la vitamine PP, la vitamine B3, le zinc, les cyclines), de la rosacée (le perméthol, la génistéine, l'esculoside, le sulfate de dextran, l'hespéridine méthylchalcone, les rétinoïdes, la licochalcone, l'oxyméthazoline, la kinétine, l'extrait de réglisse, les polyphénols, les flavonoïdes, les procyanidols (thé vert..), la vitamine P like, l'extrait de petit houx, le *Sophora japonica,* l'extrait d'Hamamélis, les antibiotiques tes que la doxycycline) ou du psoriasis (les corticoïdes, le calcipotriol, le calcitriol, le tazarotène, l'huile de cade, l'acitrétine, la PUVA thérapie).
Les immnumodulateurs pouvant être utilisés en association sont avantageusement le tacrolimus, le pimécrolimus et les oxazolines. Les oxazolines pouvant être utilisées en association sont avantageusement des oxazolines choisies dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100 ou Cyclocéramide®.
Les agents hypopigmentants ou dépigmentants pouvant être utilisés en association sont l'hydroquinone et ses dérivés, l'arbutine, l'acide rétinoïque, le rétinol, le rétinaldéhyde, la trétinoine, l'hydroquinone, les corticoïdes, l'acide kojique, l'acide azélaïque, l'acide ellagique, l'acide pyruvique, l'acide glycolique, la vitamine B3 (niacinamide) ou PP, la vitamine C, le Cyclocéramide®, les dérivés du résorcinol, le resvératrol, des extraits de réglisse ou de murier blanc, l'acide alpha-lipoïque, l'acide linoléique, l'indométhacine, des chélateurs de cations tels que l'EDTA (acide éthylène diamine tétra acétique), les extraits de soja tels que la génistéine. On peut également citer le Sepiwhite® (N-undecylenoyl-L-phénylalanine) commercialisé par la société Seppic, qui est un agent cosmétique dépigmentant.
Les agents pigmentants pouvant être utilisés en association sont par exemple des agents qui colorent la peau comme le dihydroxyacétone et les mélanines ; des agents qui stimulent le procédé de pigmentation naturelle comme les psolarènes ayant des propriétés thérapeutiques en dermatologie (le 8-méthoxypsolarène, le 5-méthoxypsolarène, le 4,5',8-triméthylpsolarène ou des extraits végétaux de *Psorelea corylifolia* et de *Ammi majus*), les caroténoïdes (le lycopène, la canthaxanthine), les agents stimulant la voie de l'AMP cyclique (1. les analogues de l'AMPc, tels que le 8-bromo-AMPc ou le dibutiryl-AMPc, 2. la forskoline, 3. l'isobutyl-méthyl-xanthine ou la théophylline), les activateurs des protéines kinase C (les diacylglycérols, en particulier l'oléyl-acétyl-glycérol), les diols aliphatiques ou cycliques (le 1,2-propandiol, le 5-norbomane-2,2-diméthanol, le norbomane-2,2-diméthano), les diols bicycliques monoterpène, les dérivés de tyrosine (la L-tyrosine, la L-DOPA), le diméthylsulfoxyde, les agents lysomotropiques, les dinucléotides thymidine, les fragments d'ADN, les analogues de l'hormone stimulant les mélanocytes, le 3-isobutyl-1-méthylxanthine, les donneurs d'acide nitrique (Brown, Journal of photochemistry and photobiology B : biology 63 (2001) 148-161) ; ou encore des extraits végétaux comme les peptides de riz, et les algues, démontrant une activité promélanogène : *Laminaria digitata* (Thalitan® de Codif).
Une association particulièrement avantageuse selon l'invention est une composition comprenant l'extrait peptidique et osidique de graines de *Vigna unguiculata* et des insaponifiables végétaux et animaux, comme par exemple, les insaponifiables d'Avocat et de Soja, et des concentrats d'huile végétale ou animale en insaponifiable, comme par exemple le concentrat d'huile de Tournesol ou d'huile de Palme, ou encore des huiles végétales contenant des insaponifiables comme par exemple les huiles de Soja et de Colza, et les dérivés d'insaponifiables comme les furanes d'Avocat, les insaponifiables stéroliques, les esters de stérols et les dérivés vitaminiques. Les insaponifiables « stéroliques » sont des insaponifiables dont la teneur en stérols, en méthylstérols et en alcools triterpèniques est comprise entre 20 et 95 % en poids, de préférence 45-65 % en poids, par rapport au poids total de l'insaponifiable.
Une association particulièrement avantageuse selon l'invention est une composition comprenant l'extrait de graines de *Vigna unguiculata* et des sucres d'Avocat (voir demande WO 2005/115421). Cette composition est particulièrement adaptée pour le traitement de la réparation de la barrière cutanée et de l'inflammation.
Une association particulièrement avantageuse selon l'invention est une composition comprenant l'extrait de graines *Vigna unguiculata* et des peptides d'Avocat (voir demande internationale WO2005/105123). Cette composition est particulièrement adaptée pour le traitement de l'irritation et de l'inflammation.
Une autre association particulièrement avantageuse selon l'invention est une composition comprenant l'extrait de graines de *Vigna unguiculata* et une huile d'Avocat (voir les demandes internationales WO2004/012496, WO2004/012752, WO2004/016106, WO2007/057439).
Une autre association particulièrement avantageuse selon l'invention est une composition comprenant l'extrait de graines de *Vigna unguiculata* et l'Avocadofurane® (furanes d'avocat, pouvant être obtenus par le procédé décrit dans la demande internationale WO 01/21605). Cette composition est particulièrement adaptée pour le traitement de l'inflammation, pour favoriser la cicatrisation, et pour ses propriétés anti-âge.
Une autre association particulièrement avantageuse selon l'invention est une composition comprenant l'extrait de graines de graines de *Vigna unguiculata* et le 5 alpha Avocuta® (butyl avocadate). Cette composition est particulièrement adaptée pour inhiber la 5 alpha réductase (voir WO 01/52837 et WO 02/06205) et réguler la sécrétion séborrhée se trouvant augmentée dans l'acné ou les pellicules.
Une autre association particulièrement avantageuse selon l'invention est une composition comprenant l'extrait de graines de *Vigna unguiculata* et les insaponifiables d'Avocat et de Soja. Les insaponifiables d'Avocat et de Soja pouvant être utilisés en association sont avantageusement un mélange d'insaponifiables d'Avocat furaniques et d'insaponifiables de Soja, dans un rapport respectif d'environ 1/3-2/3. Les insaponifiables d'Avocat et de Soja sont encore plus avantageusement le produit Piasclédine®, commercialisé par les Laboratoires Expanscience.
Une autre association particulièrement avantageuse selon l'invention est une composition comprenant l'extrait de graines de *Vigna unguiculata* et un oléodistillat de Tournesol, encore plus avantageusement avec des concentrats de Tournesol linoléiques, tels que l'actif commercialisé par les Laboratoires Expanscience, Soline® (cf. la demande internationale WO 01/21150), Cette composition est particulièrement adaptée pour le traitement de l'inflammation et pour la réparation de la barrière cutanée.
Une autre association particulièrement avantageuse selon l'invention est une composition comprenant l'extrait de graines de *Vigna unguiculata* et un insaponifiable de Soja, tel qu'obtenu selon le procédé décrit dans la demande internationale WO01/51596.
Une autre association particulièrement avantageuse selon l'invention est une composition comprenant l'extrait de graines de *Vigna unguiculata* et du Lupéol (FR 2 822 821, FR 2 857 596). Cette composition est particulièrement adaptée pour favoriser la cicatrisation.
Une autre association particulièrement avantageuse selon l'invention est une composition comprenant l'extrait de graines de *Vigna unguiculata* et des peptides de Lupin tels qu'obtenus selon le procédé décrit dans la demande WO2005/102259. Cette composition est particulièrement adaptée pour le traitement de l'inflammation et est utilisée pour ses propriétés anti-âge.
Une autre association particulièrement avantageuse selon l'invention est une composition comprenant l'extrait de graines de *Vigna unguiculata* et un extrait total de Lupin (voir demande internationale WO2005/102259). Cette composition est particulièrement adaptée pour le traitement des irritations.
Une autre association particulièrement avantageuse selon l'invention est une composition comprenant l'extrait de graines de *Vigna unguiculata* et une huile de Lupin, avantageusement une huile de Lupin blanc doux, telle que celle décrite dans la demande internationale WO 98/47479.
Une autre association particulièrement avantageuse selon l'invention est une composition comprenant l'extrait de graines de *Vigna unguiculata* et un extrait peptidique de Maca (voir demande internationale WO2004/112742). Cette composition est particulièrement appréciée pour ses propriétés cicatrisantes et anti-âge.
Une association particulièrement avantageuse selon l'invention est une composition comprenant l'extrait de graines peptidique et osidique de *Vigna unguiculata* et des peptides de Riz (voir demande internationale WO 2008/009709). Cette composition est particulièrement appréciée pour ses propriétés de stimulation de la mélanogénèse et du transfert de la mélanine.
Une autre association particulièrement avantageuse selon l'invention est une composition comprenant l'extrait de graines de *Vigna unguiculata* et du Cyclocéramide® (dérivé d'oxazoline) tel que décrit dans les demandes internationales WO2004050052, WO2004050079, et WO2004112741. Cette composition est particulièrement adaptée pour le traitement des réactions inflammatoires.
Une autre association particulièrement avantageuse selon l'invention est une composition comprenant l'extrait de graines de *Vigna unguiculata* et un extrait de Quinoa, en particulier un extrait peptidique (voir la demande internationale WO2008/080974). Cette composition est particulièrement adaptée pour le traitement des conditions inflammatoires et de la réparation de la barrière cutanée.
Une autre association particulièrement avantageuse selon l'invention est une composition comprenant l'extrait de graines de *Vigna unguiculata* et un beurre de Cupuaçu. Cette composition est particulièrement appréciée pour ses propriétés hydratantes.
Une autre association particulièrement avantageuse selon l'invention est une composition comprenant l'extrait de graines de *Vigna unguiculata* et un concentrat de Colza.
Une autre association avantageuse selon l'invention est une composition comprenant l'extrait de graines de *Vigna unguiculata* et un concentrat de Maïs.
Une autre association avantageuse selon l'invention est une composition comprenant l'extrait de graines de *Vigna unguiculata* et un extrait de fruit de *Schizandra sphenanthera* (voir demandes françaises FR 0955343 et FR 0955344).
Une autre association avantageuse selon l'invention est une composition comprenant l'extrait de graines de *Vigna unguiculata* et un extrait de graines d'*Acacia macrostachya.*

Toutes ces associations comprennent au moins un autre composé actif, en plus de l'extrait de graines de *Vigna unguiculata,* et peuvent comprendre deux, trois, quatre ou plus de composés actifs tels que décrits précédemment.

La composition selon l'invention peut être formulée sous la forme de différentes préparations adaptées à une administration topique, à une administration orale, rectale, vaginale, nasale, auriculaire ou bronchique, ainsi qu'à une administration parentérale.

Selon une première variante, les différentes préparations sont adaptées à l'administration topique et incluent notamment les crèmes, les émulsions, les laits, les pommades, les lotions, les huiles, les solutions aqueuses ou hydro-alcooliques ou glycoliques, les poudres, les patchs, les sprays, les shampooings, les vernis ou tout autre produit pour application externe.
Selon une deuxième variante, les différentes préparations sont adaptées à une administration orale ; l'extrait de graines de *Vigna unguiculata* pouvant entrer soit dans un complément alimentaire soit dans une composition nutraceutique. Le complément alimentaire peut se présenter sous forme de l'extrait graines de *Vigna unguiculata* en tant que tel ou bien sous forme de gélules ou de capsules molles de gélatine ou végétales dans le cadre de la présente invention. Ledit complément alimentaire peut alors contenir de 10 à 100% en poids de l'extrait de graines de *Vigna unguiculata.*

Les modes d'administration, les posologies et les formes galéniques optimales des composés et compositions selon l'invention peuvent être déterminés selon les critères généralement pris en compte dans l'établissement d'un traitement pharmaceutique, en particulier dermatologique, cosmétique ou vétérinaire adapté à un patient ou à un animal comme par exemple l'âge ou le poids corporel du patient ou de l'animal, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient pharmaceutiquement acceptable, notamment dermatologiquement acceptable, ou un excipient cosmétiquement acceptable. Selon la première variante, on utilise un excipient adapté pour une administration par voie topique externe. La composition selon la présente invention peut en outre comprendre au moins un adjuvant pharmaceutique ou cosmétique connu de l'homme du métier, choisi parmi les épaississants, les conservateurs, les parfums, les colorants, des filtres chimiques ou minéraux, les agents hydratants, les eaux thermales, etc.

La composition comprenant un extrait de graines de *Vigna unguiculata* ayant les spécifications indiquées est particulièrement destinée à une utilisation cosmétique, pharmaceutique, dermatologique ou nutraceutique.

Dans le cadre d'une utilisation cosmétique, pharmaceutique, ou dermatologique, la composition sera avantageusement formulée sous la forme d'une préparation adaptée à une administration topique. La composition comprenant un extrait peptidique et osidique est particulièrement destinée à une utilisation cosmétique, pharmaceutique, ou dermatologique.

Dans le cadre d'une utilisation à visée nutraceutique ou cosmétique («cosmet-food »), la composition sera avantageusement formulée sous la forme d'une préparation adaptée à une administration orale.

L'invention a également pour objet l'utilisation d'un extrait de graines de *Vigna unguiculata,* pour la fabrication d'une composition cosmétique, pharmaceutique, dermatologique ou d'une composition nutraceutique.

Avantageusement, la composition ou l'extrait selon la présente invention est utilisée dans la prévention et/ou le traitement des troubles ou pathologies de la peau et/ou des muqueuses et/ou des phanères. De manière particulièrement avantageuse, l'extrait ou la composition selon l'invention est utilisé dans des applications cosmétiques, avantageusement par voie topique, notamment pour le soin ou l'hygiène de la peau et/ou des muqueuses et/ou des phanères tels que les cheveux, ou encore pour la prévention et/ou le traitement des troubles de la peau et/ou des muqueuses et/ou des phanères tels que les cheveux.

La composition ou l'extrait selon la présente invention peut également être avantageusement utilisée dans la prévention et/ou le traitement des troubles vasculaires.

La composition ou l'extrait selon la présente invention peut également être avantageusement utilisée dans la prévention et/ou le traitement des altérations du tissu adipeux.

En particulier, la composition ou l'extrait selon l'invention est destiné à la prévention et/ou au traitement des réactions ou pathologies allergiques, inflammatoires, irritatives ou des troubles de la barrière ou de l'homéostasie de la peau, des phanères (cheveux et ongles) et/ou des muqueuses (gencives, parodontes, muqueuses génitales) immature(s), normale(s) ou mature(s)/âgée(s).

Avantageusement, la composition ou l'extrait selon l'invention peut être utilisé(e) pour la prévention et/ou le traitement des réactions, troubles ou pathologies :
- de la peau, telles que l'acné, la rosacée ou érythrocouperose, le psoriasis, les troubles vasculaires, la dermite du siège, la dermatite atopique, l'eczéma, la dermatite de contact, la dermatite irritative, la dermatite allergique, la dermite séborrhéique (croûte de lait), le psoriasis, la peau sensible, la peau réactive, la peau sèche (xérose), la peau déshydratée, la peau avec rougeur, l'érythème cutanée, la peau âgée ou photoâgée, la peau photosensibilisée, la peau pigmentée (mélasma, pigmentation post-inflammatoire...), la peau dépigmentée (vitiligo), la peau avec cellulite, la peau relâchée, la peau avec vergetures, les dartres, les gerçures, les piqûres, les crevasses en particulier des seins, les coups de soleil, les inflammations dus aux rayons de toutes sortes, les irritations par agents chimiques, physiques (par exemple contrainte de tension pour les femmes enceintes), bactériologiques, fongiques ou viraux, parasitaires (poux, gale, teigne, acariens, dermatophytes), radiologiques ou par déficit de l'immunité innée (peptides antimicrobiens) ou acquise (cellulaire, humorales, cytokines), et/ou
- des muqueuses telles que les gencives et parodontes pouvant présenter des gingivites (gencives sensibles des nouveaux nés, problèmes d'hygiène, dus au tabagisme ou autres), des parodontopathies, ou des muqueuses génitales pouvant présenter des irritations des sphères génitales males ou femelles externes ou internes et/ou
- des phanères tels que les ongles (ongles cassants, fragiles ...) et des cheveux (alopécie, pellicules, hirsutismes, dermites séborrhéiques, folliculites) immatures, normaux ou matures, présentant en particulier des troubles du cuir chevelu tels que les alopécies (ou pelade) androgénétiques, aiguës, localisées, cicatricielles, congénitales, occipitales du nourrisson, aerata, dues à la chimiothérapie/radiothérapie ou encore l'effluvium télogène, l'effluvium anagène, la dystrophie pilaire, la trichotillomanie, la teigne ou les pellicules grasses ou sèches.

Avantageusement, la composition ou l'extrait selon l'invention peut être utilisé(e) pour la prévention et/ou le traitement des inflammations.

L'invention concerne également un procédé de soin cosmétique de la peau et/ou des phanères et/ou des muqueuses, en vue d'améliorer leur état et/ou leur aspect, comprenant l'administration ou consistant à administrer une composition ou un extrait selon la présente invention.

De manière particulièrement avantageuse, la présente invention concerne l'utilisation cosmétique de la composition ou l'extrait pour restaurer les troubles ou désordres de la barrière cutanée, pour augmenter la synthèse des lipides épidermiques, pour renforcer la fonction barrière de la peau, notamment pour lutter contre le stress ou les agressions environnementales ou les agressions ou irritations chimiques provoquées par exemple par les médicaments ou encore les agressions par des micro-organismes, sur la peau, les muqueuses ou les phanères, notamment les cheveux.

En particulier, la composition ou l'extrait est utilisé, avantageusement dans des applications cosmétiques, pour hydrater la peau ou les muqueuses, pour traiter les peaux sèches, les peaux atopiques, les peaux acnéiques, les peaux irritées/inflammées, les peaux allergiques, les peaux avec rougeurs, les peaux avec dermite séborrhéique, les peaux agressées, les peaux sensibles, les peaux réactives, les peaux photo-sensibilisées, les peaux âgées ou photo-âgées, de manière générale comme agents antivieillissement de la peau (vieillissement intrinsèque ou extrinsèque), notamment comme agents photo-vieillissement ou agents anti-UV, comme agents pro-pigmentant, pour cicatriser la peau, comme agents anti-oxydants et anti-microbiens, ou encore pour le soin des cheveux, des ongles ou des muqueuses.

### EXEMPLES

### Exemple 1 : Compositions pour application par voie topique

Plusieurs compositions pour application par voie topique sont présentées ci-dessous. L'extrait peptidique et osidique de *Vigna unguiculata* peut être incorporé à divers produits cosmétiques tels que des eaux nettoyantes, des émulsions huile dans eau, des émulsions eau dans huile, des huiles, des laits, des lotions, des shampooings, des produits moussants et sprays, dont les compositions sont présentées ci-dessous à titre d'exemples.

### EAU NETTOYANTE PEAU SENSIBLE

| **Matière première / Nom commercial ou Nom INCI** | **%** |
|---|---|
| CAPRYLOYL GLYCINE | De 0 à 1 % |
| LESSIVE SOUDE | De 0 à 1 % |
| SEQUESTRANT | De 0 à 1 % |
| BUTYLENE GLYCOL | De 1 à 5 % |
| BETA CAROTENE | De 0 à 2 % |
| EXTRAIT DE VIGNA UNGUICULATA | De 0,001 à 10 % |
| CONSERVATEURS | De 0 à 1 % |
| PEG-32 | De 1 à 5 % |
| PEG-7 PALMCOCOATE | De 1 à 5 % |
| GLUCONATE ZINC | De 0 à 1 % |
| ACIDE CITRIQUE | De 0 à 1 % |
| EAU PURIFIÉE | QSP 100 % |
| PARFUM | De 0 à 1 % |
| POLOXAMER 184 | De 1 à 5 % |

### EMULSION ANTI-AGE

| **Matière première / Nom commercial ou Nom INCI** | **%** |
|---|---|
| ISOPARAFFINE LIQUIDE | De 5 à 20 % |
| STEARATE D'ISOCETYLE | De 5 à 20 % |
| HYDROXYSTEARATE AL - MG | De 5 à 20 % |
| ABIL WE 09 | De 1 à 5 % |
| GLYCEROL | De 1 à 5 % |
| HUILE VASELINE | De 1 à 5 % |
| ZINC OXYDE MICRONISE | De 1 à 5 % |
| BUTYLENE GLYCOL | De 1 à 5 % |
| RETINOL | De 0 à 1 % |
| VITAMINE C | De 0 à 5 % |
| EXTRAIT DE VIGNA UNGUICULATA | De 0,01 à 10 % |
| ISONONYL ISONONANOAT | De 1 à 5 % |
| CIRE D'ABEILLE | De 1 à 5 % |
| TARTRATE DE SODIUM | De 1 à 5 % |
| CHLORURE DE SODIUM | De 0 à 5 % |
| GLYCINE | De 1 à 5 % |
| CONSERVATEURS | De 0 à 1 % |
| CHOLESTEROL | De 0 à 1 % |
| PHYTOSPHINGOSINE | De 0 à 1 % |
| ACIDE TARTRIQUE | De 0 à 1 % |
| EAU PURIFIÉE | QSP 100 % |

### LAIT pour PEAU SECHE, ATOPIQUE

| **Matière première / Nom commercial ou Nom INCI** | **%** |
|---|---|
| HUILE AMANDE DOUCE | De 1 à 5 % |
| HUILE MAIS | De 1 à 5 % |
| ACIDE STEARIQUE | De 1 à 5 % |
| ALCOOL CETYLIQUE C16 C18 | De 0 à 1 % |
| ANTIMOUSSE 70414 | De 0 à 1 % |
| ALCOOL LAURIQUE 11OE | De 1 à 5 % |
| MONOLAURATE PEG 300 | De 0 à 1 % |
| MONOLEATE DE GLYCEROL | De 0 à 1 % |
| MONOSTEARATE DE GLYCEROL | De 1 à 5 % |
| VITAMINE B12 | De 0 à 5 % |
| EXTRAIT DE VIGNA UNGUICULATA | De 0,1 à 10 % |
| CONSERVATEURS | De 0 à 1 % |
| ACIDE CITRIQUE | De 0 à 1 % |
| CITRATE TRISODIQUE | De 0 à 1 % |
| EAU PURIFIEE | QSP 100 % |
| PARFUM | De 0 à 1 % |
| HUILE ARACHIDE | De 1 à 5 % |
| HUILE PALMISTE HYDROGENEE | De 1 à 5 % |

### SPRAY APAISANT

| **Matière première / Nom commercial ou Nom INCI** | **%** |
|---|---|
| EAU PURIFIÉE | QSP 100 % |
| TRILAURETH-4 PHOSPHATE | De 1 à 5 % |
| DICAPRYLYL CARBONATE | De 1 à 5 % |
| BUTYLENE GLYCOL | De 1 à 5 % |
| ERYTHRITYL ESTER | De 1 à 5 % |
| HUILE VASELINE FLUIDE | De 1 à 5 % |
| BEURRE DE KARITE | De 0 à 1 % |
| HUILE VEGETALE | De 0 à 1 % |
| CONSERVATEURS | De 0 à 1 % |
| LYCOPENE | De 0 à 5 % |
| EXTRAIT DE VIGNA UNGUICULATA | De 0,01 à 10 % |
| LESSIVE SOUDE | De 0 à 1 % |
| PARFUM | De 0 à 1 % |
| GOMME XANTHANE | De 0 à 1 % |
| CARBOPOL | De 0 à 1 % |
| SEQUESTRANT | De 0 à 1 % |
| ACIDE CITRIQUE | De 0 à 1 % |

### EMULSION ANTI-ACNEIQUE

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| VITAMINE E | De 0 à 1 % |
| VITAMINE B3 | De 0 à 5 % |
| ACIDE LINOLEIQUE | De 0 à 1 % |
| EXTRAIT DE VIGNA UNGUICULATA | De 0,01 à 10 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

### EMULSION ANTI-ROUGEURS

| **Matière première / Nom commercial ou Nom INCI** | **%** |
|---|---|
| PEG 40 STEARATE | De 1 à 5 % |
| PEG 5 GLYCERYL STEAR | De 1 à 5 % |
| CERESINE WAX | De 1 à 5 % |
| MONOSTEARATE DE GLYCEROL | De 1 à 5 % |
| STEARATE DE SORBITAN | De 0 à 2 % |
| ALCOOL CETYLIQUE | De 0 à 2 % |
| ALCOOL DI-MALATE | De 5 à 20 % |
| ESCULOSIDE | De 0 à 2 % |
| SOPHORA JAPONICA | De 0 à 5 % |
| VITAMINE E | De 0 à 1 % |
| EXTRAIT DE VIGNA UNGUICULATA | De 0,01 à 10 % |
| BUTYLENE GLYCOL | De 1 à 5 % |
| PIROCTOLAMINE | De 0 à 1 % |
| CONSERVATEURS | De 0 à 1 % |
| GLYCEROL | De 1 à 10 % |
| GOMME XANTHANE | De 0 à 1 % |
| ZINC PCA | De 0 à 2 % |
| AMIDON DE RIZ | De 1 à 5 % |
| NYLON 6 | De 0 à 2 % |
| POLYACRYLAMIDE GEL | De 1 à 5 % |
| VITAMINE B6 | De 0 à 1 % |
| PARFUM | De 0 à 1 % |
| EAU PURIFIÉE | QSP 100 % |

### SHAMPOOING ANTIPELLICULAIRE

| **Matière première / Nom commercial ou Nom INCI** | **%** |
|---|---|
| EAU PURIFIÉE | QSP 100 % |
| LAUROAMPHOACETATE | De 5 à 20 % |
| COCOGLUCOSIDE | De 5 à 20 % |
| DISTEARATE DE PEG 6000 | De 1 à 5 % |
| CONSERVATEURS | De 0 à 2 % |
| VITAMINE F | De 0 à 5 % |
| PIROCTONE OLAMINE | De 0 à 2 % |
| EXTRAIT DE VIGNA UNGUICULATA | De 0,01 à 10 % |
| ZINC PYRITHIONE | De 0 à 1 % |
| AJUSTEUR pH | De 0 à 1 % |
| SEQUESTRANT | De 0 à 1 % |
| PARFUM | De 0 à 1 % |

### SPRAY SOLAIRE SPF 50+

| **Matière première / Nom commercial ou Nom INCI** | **%** |
|---|---|
| CAPRYLO CAPRATE DE GLYCEROL | De 5 à 20% |
| CYCLOPENTASILOXANE | De 10 à 20% |
| DICAPRYLYL CARBONATE | De 5 à 20% |
| TINOSORB S | De 1 à 10% |
| OXYDE DE TITANE 100 | De 10 à 20% |
| HECTORITE | De 0 à 5% |
| ALPHA TOCOPHEROL | De 0 à 2% |
| LAURYLGLUCOSIDE-GLYSTEARATE | De 0 à 10% |
| EAU PURIFIÉE B4 | QSP 100% |
| ACIDE CITRIQUE | De 0 à 2% |
| PENTYLENE GLYCOL | De 0 à 5% |
| GLYCEROL | De 0 à 5% |
| GOMME XANTHANE | De 0 à 2% |
| EXTRAIT DE VIGNA UNGUICULATA | De 0,01 à 10 % |
| ALOE VERA | De 0 à 1% |
| GLUCONATE ZINC | De 0 à 1% |
| CONSERVATEURS | De 0 à 2% |
| TINOSORB M | De 1 à 10% |

### Exemple 2 : Compositions pour administration par voie orale

Les extraits de Vigna unguiculata peuvent être avantageusement intégrés à des compositions orales, typiquement dans des compositions permettant l'administration de 50 mg à 200 mg d'extrait de *Vigna unguiculata* par jour.

### 1/ Composition anti-vergetures sous forme de capsules molles

- EXTRAIT DE VIGNA UNGUICULATA 30 mg
- Huile d'Awara 60 mg
- Huile de colza riche en insaponifiable 300 mg
- Vitamine du groupe B (B1, B2, B3, B5, B6, B9, B12), QSP 100% des AJR
- Tocotriénols QSP 50 % AJR
- Vitamine E
- Cire d'abeille
- Lécithine de soja
- Gélatine alimentaire
- Glycérine QSP 1 capsule molle Cette composition est administrée de 4 à 6 capsules de 500 mg par jour.

### 2/ Comprimés anti-chute cheveux

- EXTRAIT DE VIGNA UNGUICULATA 25 mg
- Extraits de céréales (blé, sarrasin, millet, épeautre) riche en acides aminés soufrés 200 mg
- Vitamine C QSP 50 % des AJR
- Glycosaminoglycanes issus de cartilages de poissons 200 mg
- Glucidex IT 19 (agent de compression) QSP
   1 comprimé de 800 mg.

Cette composition est administrée de 5 à 8 comprimés par jour.

### 3/ Exemples en stick poudre amincissant

- EXTRAIT DE VIGNA UNGUICULATA 100 mg
- Extrait de thé riche en polyphénols 100 mg
- Extrait de raisin riche en OPC 50 mg
- Bétaglucanes d'origine végétale 100 mg
- Gomme xanthane 1 mg
- Ascorbate de sodium 0,3 mg
- Maltodextrine QSP 5 g.

Cette composition est administrée 2 fois par jour.

### 4/ Exemple en stick poudre anti-âge

- **EXTRAIT DE VIGNA UNGUICULATA 100 mg**
- Extrait de *Centella asiatica* 100 mg
- Magnésium, sélénium, manganèse QSP 100 % des AJR.
- Gomme xanthane 1 mg
- Ascorbate de sodium 0,3 mg
- Maltodextrine QSP 5 g.

Cette composition est administrée 2 fois par jour.

### Exemple 3 : Tests d'activités biologiques de l'extrait selon l'invention

L'extrait peptidique et osidique de Vigna unguiculata, qui est un extrait préparé par hydrolyse enzymatique, est nommé ci-après dans cet exemple hydrolysat de Vigna.

### A. Screening préliminaire d'activité sur épidermes reconstruits

Les activités biologiques de l'hydrolysat de Vigna ont été évaluées par un test de modulation d'expression de gènes sur épidermes reconstruits. Ainsi, l'expression de 64 gènes d'intérêts majeurs en physiologie cutanée et cosmétique a été étudiée par PCR-array sur des épidermes reconstruits en cours de différenciation.

### a. Matériel et Méthodes :

L'hydrolysat de Vigna (0,05% et 0,1%, p/v) a été ajouté dans le milieu de culture d'épidermes reconstruits à J5. Ceux-ci ont alors été incubés pendant 48 heures.

L'expression des marqueurs sélectionnés a été évaluée par RT-PCR quantitative (PCR-array).

La variation d'expression des marqueurs étudiés par rapport au témoin a été exprimée en pourcentage (Témoin : 100%).

### b. Résultats :

Les résultats les plus significatifs sont présentes dans le tableau ci-dessous et tendent à montrer que l'hydrolysat de Vigna, en faisant varier l'expression génique de certains marqueurs, a un intérêt particulier notamment dans les activités suivantes :
- **L'hydratation :** ä Claudine 1, CD44, cornuline.
- **La différenciation épidermique et la fonction barrière** : ä calmoduline, caténine, desmogléine 1, kératine 1, kératine 10, filaggrine, loricrine, sulfotransférase, cornuline, small prolin-rich protéines.
- **La synthèse de lipides épidermiques** : ä fatty acid synthase, glucocérébrosidase.
- **La cicatrisation** : ä calmoduline, MMP9, S 100A7.
- **Les défenses anti-microbiennes** : ä Beta-défensine, Peptidase Inhibitor 3, RNase 7, S100A7, cathélicidine.
   - - **Les défenses anti-oxydantes** : ä hème-oxygénase 1, HSP27.

### Variations de l'expression de gènes d'intérêt dans des épidermes reconstruits.

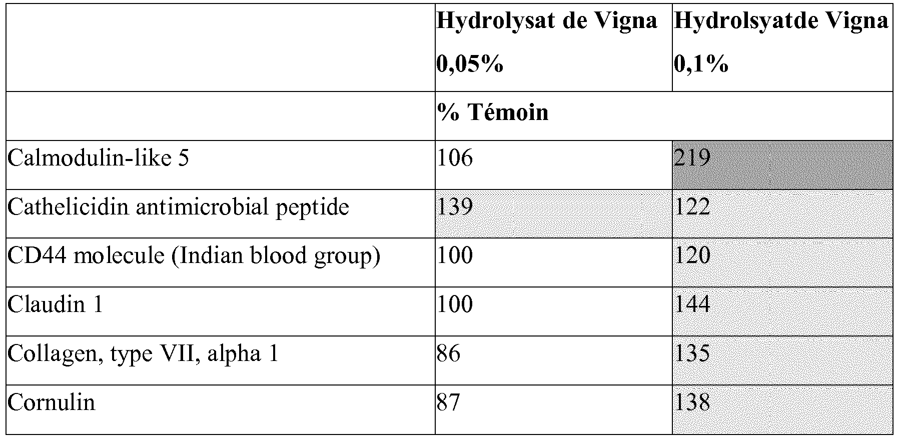

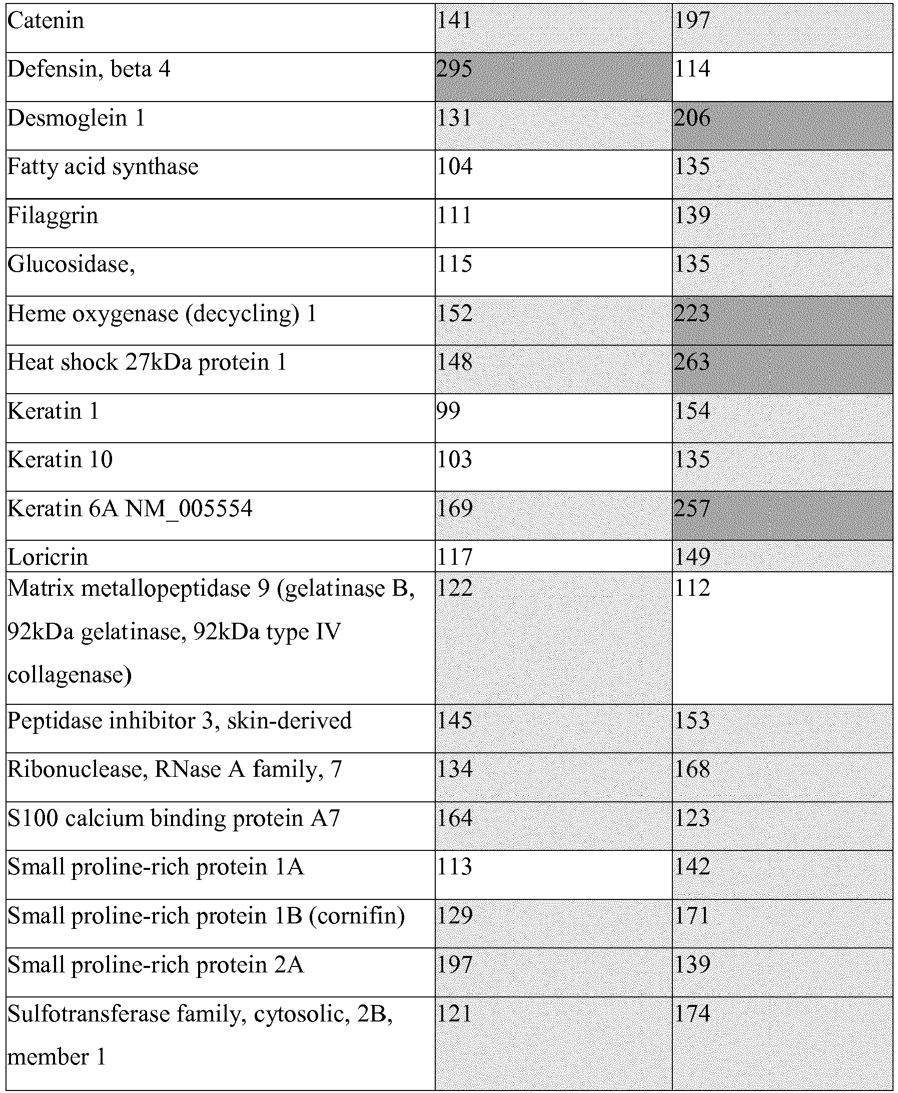

### Légendes :

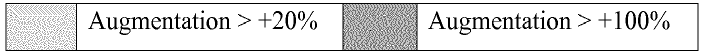

### c. Conclusion :

Ainsi, à l'issue de ce screening, l'activité de l'hydrolysat de Vigna a été évaluée dans les deux grands axes suivants : **défenses** (vis-à-vis du stress oxydant, du stress inflammatoire, des UV) et **réparation cutanée** (redensification du derme, cicatrisation, relipidation).

### B. Evaluation de l'activité anti-inflammatoire

### a. Introduction:

Au niveau de la peau, les kératinocytes sont les premières cellules à être activées lors d'une agression de l'environnement. Le kératinocyte « agressé» va alors initier la réaction inflammatoire en relargant des cytokines primaires (comme l'interleukine 1α ou IL1α) qui vont par la suite stimuler la production de médiateurs secondaires (comme l'interleukine 8 ou IL8).

L'effet anti-inflammatoire de l'hydrolysat de Vigna a été évalué par mesure des cytokines (IL1α, IL8) produites par des kératinocytes en réponse à une stimulation par le PMA.

### b. Matériel et Méthodes:

Des kératinocytes humains (lignée NCTC2544) ont été pré-incubés pendant 24 heures par l'hydrolysat de Vigna à 0,05% (p/v) ou la molécule de référence (dexaméthasone à 10⁻⁷M). Les cellules ont ensuite été traitées par du PMA à 0,1 tg/ml, toujours en présence d'hydrolysat de Vigna ou de dexaméthasone, pendant 24 heures. Les quantités d'IL1α et d'IL8 libérées dans le milieu de culture ont été évaluées par dosage ELISA. Les résultats ont été analysés statistiquement par une analyse de variance (ANOVA) à un facteur suivie d'un test de Tuckey :
ns p>0,05 : non significatif
* 0,01<p<0,05 : significatif
** 0,001<p<0,01 : très significatif
***p<0,001 : hautement significatif

Le pourcentage d'inhibition par rapport au témoin stimulé (PMA) a été calculé.

### c. Résultats :

L'hydrolysat de Vigna à 0,05% a significativement inhibé la libération par des kératinocytes des cytokines inflammatoires IL1α et IL8 induite par une stimulation au PMA.

### Libération de cytokines par des kératinocytes.

| | **IL1**α **(pg/ml)** | | **IL8 (ng/ml)** |
|---|---|---|---|
| Contrôle | 7,8 ± 0,0 | | 0,1 ± 0,0 |
| **Témoin stimulé (PMA)** | 42,3 ± 4,5 | (***) | 30,6 ± 1,9 (***) |
| Référence (Dexaméthasone 10⁻⁷M) | 14,0 ± 3,5 | (-82% ***) | 6,0 ± 0,2 (-81% ***) |
| **Hydrolysat de Vigna 0,05%** | **25,3 ± 4,6** | (-**49**% ***)** | **23,9 ± 1,0 (-22% *)** |

### C. Evaluation de l'effet protecteur vis-à-vis du stress oxydant

### a. Introduction:

Différents facteurs exogènes, comme par exemple les radiations UV, peuvent induire un stress oxydatif qui est susceptible de conduire à des dommages cellulaires, à un déséquilibre du statut redox ou encore à une perte de fonctions moléculaires.
Pour se protéger contre ce stress oxydatif, la cellule dispose de différents mécanismes de défenses dont font partie l'enzyme microsomale heme-oxygénase ou les HSP (Heat Shock Proteins ou Protéines de choc thermique), comme l'HSP27, qui exerce également un rôle protecteur dans les kératinocytes en régulant la production de médiateurs pro-inflammatoires.

Un modèle de fibroblastes irradiés par les UVA a été utilisé afin de rechercher un effet protecteur de l'hydrolysat de Vigna vis-à-vis de ces mécanismes de défense de la cellule contre le stress oxydant.

### b. Matériel et Méthodes:

Des fibroblastes humains normaux ont été pré-traités pendant 24 heures par l'hydrolysat de Vigna à 0,05% (p/v). Les cellules ont ensuite été irradiées par 50 kJ/m² d'UVA, puis remises à incuber en présence d'hydrolysat de Vigna pour 6 heures. L'expression génique de l'heme-oxygénase 1 et de l'HSP27 a été analysée par RT-PCR quantitative en temps réel.
Les résultats ont été analysés statistiquement par une analyse de variance (ANOVA) à un facteur suivie d'un test de Tukey :
ns p>0,05 : non significatif
* 0,01<p<0,05 : significatif
** 0,001<p<0,01 : très significatif
***p<0,001 : hautement significatif

Le niveau d'expression génique a été exprimé en quantité relative (QR) et l'effet protecteur a été évalué par le pourcentage d'inhibition par rapport au témoin stimulé (UVA).

### c. Résultats:

L'hydrolysat de Vigna a significativement inhibé l'expression génique des marqueurs 20 du stress oxydant hème-oxygénase 1 et HSP27 induits par l'irradiation aux UVA de fibroblastes.

Ainsi, l'hydrolysat de Vigna protège les cellules du stress induit par les UVA.

### Expression génique de l'hème oxygénase 1 et de l'HSP27 par des fibroblastes.

| | **HEME OXYGENASE 1** | **HSP27** |
|---|---|---|
| Cellules contrôles | 1 | 1,00 |
| Témoin UVA 50kJ/m² | **156 (***)** | **4,84 (***)** |
| **Hydrolysat de Vigna 0,05 % + UVA** | **62 (-60% *)** | **2,38 (-51% *)** |

### D. Evaluation de l'effet sur la mélanogénèse

### a. Introduction:

La production de mélanine par les mélanocytes en réponse à une exposition aux UV constitue un système de défense adaptatif de la peau. En effet, situé dans la couche basale de l'épiderme, chaque mélanocyte protége environ 36 kératinocytes.

L'effet de l'hydrolysat de Vigna a été évalué sur la production de mélanine par des mélanocytes humains normaux.

### b. Matériel et Méthodes :

Des mélanocytes épidermiques humains normaux ont été traités pendant 240 heures par un analogue stable de l'α-MSH (NDP-MSH) en présence, ou non (témoin), de l'hydrolysat de Vigna à 0,01% (p/v). La quantité de mélanine dans les mélanocytes a été évaluée par lecture de densité optique.

Les résultats ont été analysés statistiquement par une analyse de variance (ANOVA) à un facteur suivie d'un test de Dunnett :
ns p>0,05 : non significatif
* 0,01<p<0,05 : significatif
** 0,001<p<0,01 : très significatif
***p<0,001 : hautement significatif

Le pourcentage de stimulation par rapport au contrôle ou au témoin stimulé a été calculé.

### c. Résultats :

L'hydrolysat de Vigna présente un effet pro-pigmentant contribuant à renforcer la photoprotection. En effet, l'actif a potentialisé l'effet du NDP-MSH sur la mélanogénèse en stimulant significativement la production de mélanine par des mélanocytes humains normaux cultivés en présence de NDP-MSH.

### Production de mélanine par des mélanocytes humains normaux

| | **Mélanine (µg/ml)** | Stimulation par rapport au contrôle | Stimulation par rapport au témoin stimulé |
|---|---|---|---|
| Contrôle | 37,0 ± 0,3 | | |
| Témoin stimulé (NDP-MSH) | **45,5 ± 2,0** | **+23% **** | |
| **Hydrolysat de Vigna 0,01%** | **53,6 ± 1,3** | **+45%***** | **+18%** * |

### E. Evaluation de l'effet sur l'expression des protéines de la matrice dermique

### a. Introduction:

Les modifications de la peau avec l'âge résultent de modifications des fonctions cellulaires et de modifications progressives de la composition et de la structure de la matrice-extracellulaire dermique. En effet, le derme subit une perte graduelle de son épaisseur avec une diminution de macromolécules essentielles, comme le collagène et l'élastine. Cette diminution peut être attribuée à une diminution de leur synthèse et/ou à une augmentation de leur dégradation par les MMP (métalloprotéinases matricielles) par exemple.

L'effet de l'hydrolysat de Vigna sur la modulation de la matrice extra-cellulaire du derme a été évalué sur un modèle de fibroblastes dermiques sur lesquels l'expression génique de l'élastine et de la MMP1 a été étudiée.

### b. Matériel et méthodes:

Des fibroblastes humains normaux ont été traités pendant 24 heures par l'hydrolysat de Vigna à 0,01% et 0,05% (p/v) ou par le TGFβ1 à 5 ng/ml (référence). L'expression génique de l'élastine et de la MMP1 a été analysée par RT-PCR quantitative en temps réel.

Les résultats ont été analysés statistiquement par une analyse de variance (ANOVA) à un facteur suivie d'un test de Dunnett :
ns p>0,05 : non significatif
* 0,01<p<0,05 : significatif
** 0,001<p<0,01 : très significatif
***p<0,001 : hautement significatif

Le niveau d'expression génique a été exprimé en quantité relative (QR) et l'effet du traitement par rapport aux cellules contrôles en pourcentage d'augmentation ou d'inhibition.

### c. Résultats:

L'hydrolysat de Vigna a significativement stimulé l'expression génique de l'élastine et siginficativement inhibé l'expression génique de la MMP1.

Ces effets sont en faveur d'une redensification de la matrice dermique et d'une limitation de sa dégradation au cours des processus de réparation cutanée ou du vieillissement.

### Expression génique de marqueurs de la matrice dermique par des fibroblastes.

| | **ELASTINE** | **MMP1** |
|---|---|---|
| Cellules contrôles | 1,00 | 1,00 |
| Référence (TGFβ1) | **6,59 (+559% ***)** | **0,12 (-88% ***)** |
| **Hydrolysat de Vigna 0,01%** | **1,69 (+69% **)** | **0,77 (-23% **)** |
| **Hydrolysat de Vigna 0,05%** | **1,67 (+67% **)** | **0,59 (-41% ***)** |

### F. Evaluation de l'effet sur la fonction barrière

### 1. Introduction

La mise en place de la fonction barrière est liée à la différenciation épidermique qui aboutit à la formation du *stratum corneum* (SC). Différentes structures permettent au SC d'assurer sa fonction de barrière : les lipides intercellulaires, l'enveloppe cornifiée, les cornéodesmosomes.

L'effet de l'hydrolysat de Vigna sur la différenciation épidermique et le renforcement de la fonction barrière a été étudié en évaluant l'expression génique de marqueurs de la différenciation épidermique dans des kératinocytes d'une part, et en évaluant la néosynthèse des lipides dans un modèle d'épiderme reconstruit d'autre part.

### 2. Evaluation de l'expression génique de marqueurs de la différenciation épidermique

### a. Matériel et méthodes:

Des kératinocytes épidermiques humains normaux ont été traités pendant 24 heures par l'hydrolysat de Vigna à 0,01 et 0,05% (p/v) ; en parallèle des kératinocytes témoins ont été cultivés en conditions favorables à la différenciation (milieu supplémenté en calcium « High Ca »). L'expression génique des marqueurs d'intérêts sélectionnés a été analysée par RT-PCR quantitative en temps réel.

Les résultats ont été analysés statistiquement par une analyse de variance (ANOVA) à un facteur suivie d'un test de Dunnett :
ns p>0,05 : non significatif
* 0,01<p<0,05 : significatif
** 0,001<p<0,01 : très significatif
***p<0,001 : hautement significatif

Le niveau d'expression génique a été exprimé en quantité relative (QR) et l'effet du traitement par rapport aux cellules contrôles en pourcentage d'augmentation.

### b. Résultats:

L'hydrolysat de Vigna a significativement augmenté l'expression génique de l'involucrine et de la transglutaminase 1 par les kératinocytes. L'hydrolysat de Vigna a donc un effet favorable à la différenciation épidermique et à la formation de l'enveloppe cornée.

### Expression génique de marqueurs de la différenciation par des kératinocytes.

| | **INVOLUCRINE** | **TRANSGLUTAMINASE 1** |
|---|---|---|
| Cellules contrôles | 1,00 | 1,00 |
| **Hydrolysat de Vigna 0,01%** | **2,14(+114% ***)** | **1,95(+95% **)** |
| **Hydrolysat de Vigna 0,05%** | **1,61 (+61% **)** | **1,58 (+58% *)** |
| Témoin High Ca | **1,49 (+49% **)** | **2,25 (+125% ***)** |

### 3. Evaluation de l'effet sur la néosynthèse des lipides épidermiques

### a. Matériel et méthodes:

Des épidermes reconstruits de 3 jours ont été placés en milieu de différenciation contenant ou non (témoin) l'hydrolysat de Vigna à 0,01% et 0,05% (p/v) et supplémenté avec du [14C]-acétate. Les épidermes ont ensuite été incubés pendant 48 heures. Après incubation, les épidermes ont été traités à l'identique en absence d'acétate puis incubés pendant 48 heures supplémentaires.

A la fin de l'incubation, la néosynthèse des lipides a été étudiée par mesure des phospholipides et lipides neutres néoformés après chromatographie en couche mince.

### b. Résultats:

L'hydrolysat de Vigna a stimulé la synthèse de cholestérol et d'acides gras libres par les épidermes reconstruits.

### Analyse quantitative profil lipidique des lipides neutres et acides gras (% du témoin)

| | **Cholestérol** | **Acides gras libres** |
|---|---|---|
| Témoin | 100 | 100 |
| **Hydrolysat de Vigna 0,01%** | **125** | **123** |
| **Hydrolysat de Vigna 0,05%** | **130** | **130** |

### G. Conclusion

Les résultats présentés dans ce rapport ont permis de démontrer l'activité de l'extrait (hydrolysat) de Vigna selon l'invention dans deux axes distincts mais cependant complémentaires:

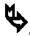 **Renforcement des défenses cutanées:**

- **Défense anti-microbienne** : stimulation de l'expression génique de peptides anti-microbiens (β-défensives, RNase7, cathélicidine...) par des épidermes reconsrtuits [*Screening préliminaire d'activité*].
- **Défenses anti-oxydantes** : stimulation de l'expression génique de l'HSP27 et l'hème-oxygénase 1 dans des épidermes reconstruits en conditions basales [*Screening préliminaire d'activité*] ; protection contre un stress UV-induit sur fibroblastes (modulation de la sur-expression UV-induite de l'HSP27 et de l'hème oxygénase 1).
- **Protection anti-UV :** effet potentialisateur de la mélanogénèse sur des mélanocytes.

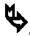 **Stimulation des processus de réparations cutanées:**

- **Action en profondeur:** stimulation de l'élastine, inhibition de la MMP1 sur fibroblastes dermiques pour une redensification de la matrice dermique.
- **Cicatrisation:** stimulation de l'expression génique de marqueurs impliqués dans la cicatrisation épidermique (calmoduline, MMP9, S100A7) dans des épidermes reconstruits [*Screening préliminaire d'activité*].

### - Reconstruction de l'épiderme et mise en place de la fonction barrière :

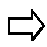 *Action sur la différenciation épidermique*: stimulation de l'expression génique de marqueurs de la différenciation et de la barrière (involucrine, transglutaminase 1) sur kératinocytes humains normaux et sur épidermes reconstruits [*Screening préliminaire d'activité*]*.*
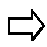 *Relipidation en surfacé*: stimulation de l'expression génique des enzymes impliquées dans la synthèse des lipides épidermiques (fatty acid synthase, glucocérébrosidase) sur épidermes reconstruits [*Screening préliminaire d'activité*] ; stimulation de la néosynthèse des lipides épidermiques (acides gras libres, cholestérol) sur épidermes reconstruits.

Ces diverses actions sont encore renforcées par une **activité anti-inflammatoire** de l'extrait (hydrolysat) de Vigna selon l'invention: modulation du relargage de cytokines inflammatoires (IL1α, IL8) sur kératinocytes vis-à-vis d'un stress chimique; induction de l'expression génique de l'HSP27 (rôle protecteur) dans des épidermes reconstruits [*Screening préliminaire d'activité*].

## Revendications

1. Composition comprenant un extrait peptidique et osidique de graines de *Vigna unguiculata* substantiellement débarrassé de toute protéine native résiduelle et le cas échéant un excipient approprié, **caractérisée en ce que** l'extrait peptidique et osidique comprend 10 à 90% en poids de peptides et 10 à 90% en poids de sucres, les pourcentages étant exprimés par rapport au poids total dudit extrait peptidique et osidique.

2. Composition selon la revendication 1, **caractérisée en ce que** l'extrait peptidique et osidique comprend 10 à 50%, avantageusement 20 à 45%, typiquement 30% à 40%, en poids de peptides.

3. Composition selon la revendication 1 ou 2, **caractérisée en ce que** l'extrait peptidique et osidique comprend 20 à 80%, avantageusement 30 à 70%, typiquement 50 à 60%, en poids de sucres.

4. Composition selon l'une quelconque des revendications précédentes comprenant au moins un autre composé actif en plus de l'extrait de graines de *Vigna unguiculata.*

5. Composition selon l'une quelconque des revendications précédentes, **caractérisée en ce qu'**il s'agit d'une composition cosmétique, pharmaceutique, dermatologique ou nutraceutique.

6. Composition selon l'une quelconque des revendications précédentes, **caractérisée en ce qu'**elle est formulée pour être administrée par voie topique ou orale.

7. Procédé de préparation d'un extrait peptidique et osidique des graines de *Vigna unguiculata* substantiellement débarrassé de toute protéine native résiduelle comprenant les étapes successives suivantes :
- dispersion en phase aqueuse des graines broyées, avantageusement à un pH compris entre 3.0 et 9.0 et à une température comprise entre 20 et 90°C,
- hydrolyse enzymatique de ladite dispersion, réalisée par un mélange enzymatique de protéases et de carbohydrases, telles que des pectinases, cellulases, arabanases, hémicellulases, et β-glucanases, et
- récupération de l'extrait peptidique et osidique.

8. Procédé selon la revendication 7, **caractérisé en ce que**, suite à l'hydrolyse de ladite dispersion, et préalablement à la récupération de l'extrait peptidique et osidique, on procède à une étape d'ultrafiltration par exemple à un seuil de coupure compris entre 10000 et 15000 Daltons.

9. Extrait de graines de *Vigna unguiculata* substantiellement débarrassé de toute protéine native résiduelle susceptible d'être obtenu par le procédé selon l'une quelconque des revendications 7 à 8, contenant 10 à 90% en poids de peptides et 10 à 90% en poids de sucres.

10. Extrait selon la revendication 9, contenant 10 à 50 %, avantageusement 20 à 45%, typiquement 30% à 40%, en poids de peptides.

11. Extrait selon la revendication 9 ou 10, contenant 20 à 80 %, avantageusement 30 à 70 %, typiquement 50 à 60%, en poids de sucres.

12. Extrait selon l'une des revendications 9 à 11 ou composition selon l'une quelconque des revendications 1 à 6 pour son utilisation dans la prévention et/ou le traitement des troubles ou pathologies de la peau et/ou des muqueuses et/ou des phanères.

13. Utilisation non-thérapeutique cosmétique d'un extrait selon l'une des revendications 9 à 11 ou d'une composition selon l'une quelconque des revendications 1 à 6 pour la prévention et/ou le traitement des altérations du tissu adipeux.

14. Extrait selon l'une des revendications 9 à 11 ou composition selon l'une quelconque des revendications 1 à 6 pour son utilisation pour cicatriser la peau.

15. Procédé de soin non-thérapeutique cosmétique de la peau et/ou des phanères et/ou des muqueuses, en vue d'améliorer leur état et/ou leur aspect, comprenant l'administration d'une composition cosmétique telle que définie selon l'une quelconque des revendications 1 à 6 ou un extrait tel que défini selon l'une quelconque des revendications 9 à 11.

## Patentansprüche

1. Zusammensetzung, umfassend einen Peptid- und Ose-Extrakt von Samen von *Vigna unguiculata,* substantiell befreit von allen nativen Restproteinen, und gegebenenfalls einen geeigneten Hilfsstoff, **dadurch gekennzeichnet, dass** der Peptid- und Ose-Extrakt 10 bis 90 Gew.-% Peptide und 10 bis 90 Gew.-% Zucker umfasst, wobei die Prozentsätze in Bezug zum Gesamtgewicht des Peptid- und Ose-Extrakts ausgedrückt sind.

2. Zusammensetzung nach Anspruch 1, **dadurch gekennzeichnet, dass** der Peptid- und Ose-Extrakt 10 bis 50 Gew.-%, vorzugsweise 20 bis 45 Gew.-%, in typischer Weise 30 bis 40 Gew.-% Peptide umfasst.

3. Zusammensetzung nach Anspruch 1 oder 2, **dadurch gekennzeichnet, dass** der Peptid- und Ose-Extrakt 20 bis 80 Gew.-%, vorzugsweise 30 bis 70 Gew.-%, in typischer Weise 50 bis 60 Gew.-% Zucker umfasst.

4. Zusammensetzung nach einem der vorangehenden Ansprüche, umfassend mindestens eine andere aktive Verbindung zusätzlich zum Extrakt von Samen von *Vigna unguiculata.*

5. Zusammensetzung nach einem der vorangehenden Ansprüche, **dadurch gekennzeichnet, dass** es sich um eine kosmetische, pharmazeutische, dermatologische oder nutrazeutische Zusammensetzung handelt.

6. Zusammensetzung nach einem der vorangehenden Ansprüche, **dadurch gekennzeichnet, dass** sie formuliert ist, um auf topischem oder oralem Weg verabreicht zu werden.

7. Verfahren zur Herstellung eines Peptid- und Ose-Extrakts von Samen von *Vigna unguiculata,* substantiell befreit von allen nativen Restproteinen, umfassend die folgenden aufeinanderfolgenden Schritte:
- Dispergieren, in wässriger Phase, der zerkleinerten Samen, in vorteilhafter Weise bei einem pH zwischen 3,0 und 9,0 inklusive und bei einer Temperatur zwischen 20 und 90 °C inklusive,
- enzymatisches Hydrolysieren der Dispersion, durchgeführt durch ein enzymatisches Mischen von Proteasen und von Carbohydrasen wie Pectinasen, Cellulasen, Arabanasen, Hemicellulasen und β-Glucanasen, und
- Rückgewinnen des Peptid- und Ose-Extrakts.

8. Verfahren nach Anspruch 7, **dadurch gekennzeichnet, dass** nach der Hydrolyse der Dispersion und vor der Rückgewinnung des Peptid- und Ose-Extrakts ein Ultrafiltrationsschritt beispielsweise bei einer Ausschlussgrenze zwischen 10000 und 15000 Dalton inklusive durchgeführt wird.

9. *vigna unguiculata* Samenextrakt substantiell befreit von allen nativen Restproteinen, erhaltbar durch das Verfahren nach einem der Ansprüche 7 bis 8, enthaltend 10 bis 90 Gew.-% Peptide und 10 bis 90 Gew.-% Zucker.

10. Extrakt nach Anspruch 9, enthaltend 10 bis 50 Gew.-%, vorzugsweise 20 bis 45 Gew.-%, in typischer Weise 30 bis 40 Gew.-% Peptide.

11. Extrakt nach Anspruch 9 oder 10, enthaltend 20 bis 80 Gew.-%, vorzugsweise 30 bis 70 Gew.-%, in typischer Weise 50 bis 60 Gew.-% Zucker.

12. Extrakt nach einem der Ansprüche 9 bis 11 oder Zusammensetzung nach einem der Ansprüche 1 bis 6 für seine Verwendung bei der Vorbeugung und/oder der Behandlung von Störungen oder Pathologien der Haut und/oder der Schleimhäute und/oder der Hautanhangsgebilde.

13. Nicht therapeutische kosmetische Verwendung eines Extrakts nach einem der Ansprüche 9 bis 11 oder einer Zusammensetzung nach einem der Ansprüche 1 bis 6 für die Vorbeugung und/oder die Behandlung des adipösen Gewebes.

14. Extrakt nach einem der Ansprüche 9 bis 11 oder Zusammensetzung nach einem der Ansprüche 1 bis 6 für seine Verwendung zum Heilen der Haut.

15. Nicht therapeutisches kosmetisches Verfahren zur Pflege der Haut und/oder der Hautanhangsgebilde und/oder der Schleimhäute zur Verbesserung ihres Zustands und/oder ihres Aussehens, umfassend die Verabreichung einer kosmetischen Zusammensetzung nach einem der Ansprüche 1 bis 6 oder eines Extrakts nach einem der Ansprüche 9 bis 11.

## Claims

1. A composition comprising a peptide and oside extract of *Vigna unguiculata* seeds substantially free of any residual native protein and, if need be, a suitable excipient, **characterized in that** the peptide and oside extract comprises 10-90% by weight of peptides and 10-90% by weight of sugars, the percentages being expressed in relation to the total weight of said peptide and sugar extract.

2. The composition according to claim 1, **characterized in that** the peptide and oside extract comprises 10-50%, advantageously 20-45%, typically 30-40%, by weight of peptides.

3. The composition according to claim 1 or 2, **characterized in that** the peptide and oside extract comprises 20-80%, advantageously 30-70%, typically 50-60%, by weight of sugars.

4. The composition according to any one one of the preceding claims comprising at least one other active compound in addition to the *Vigna unguiculata* seed extract.

5. The composition according to any one one of the preceding claims, **characterized in that** said composition is a cosmetic, pharmaceutical, dermatological or nutraceutical composition.

6. The composition according to any one one of the preceding claims, **characterized in that** said composition is formulated to be administered topically or orally.

7. A method for preparing a peptide and oside extract of *vigna unguiculata* seeds substantially free of any residual native protein comprising the following successive steps:
- dispersion in an aqueous phase of ground seeds, advantageously at a pH between 3.0 and 9.0 and at a temperature between 20 and 90 °C,
- enzymatic hydrolysis of said dispersion, carried out by an enzymatic mixture of proteases and carbohydrases, such as pectinases, cellulases, arabanases, hemicellulases and β-glucanases, and
- recovery of the peptide and oside extract.

8. The method according to claim 7, **characterized in that**, following hydrolysis of said dispersion and before recovery of the peptide and oside extract, an ultrafiltration step with a cut-off between 10,000 Da and 15,000 Da, for example, is carried out.

9. A *vigna unguiculata* seed extract substantially free of any residual native protein able to be obtained by the method according any one according to claims 7 to 8, containing 10-90% by weight of peptides and 10-90% by weight of sugars.

10. The extract according to claim 9, containing 10-50%, advantageously 20-45%, typically 30-40%, by weight of peptides.

11. The extract according to claim 9 or 10, containing 20-80%, advantageously 30-70%, typically 50-60%, by weight of sugars.

12. The extract according to one of claims 9 to 11 or the composition according to any one of claims 1 to 6 for use in the prevention and/or treatment of disorders or pathologies of the skin and/or mucosae and/or appendages.

13. Non-therapeutic cosmetic use of an extract according to one of claims 9 to 11 or of a composition according to any one of claims 1 to 6 in the prevention and/or treatment of adipose tissue alterations.

14. The extract according to one of claims 9 to 11 or the composition according to any one of claims 1 to 6 for use to heal the skin.

15. A method for non-therapeutic cosmetic care of the skin and/or appendages and/or mucosae, in order to improve the condition and/or appearance thereof, comprising the administration of a cosmetic composition such as defined according to any one of claims 1 to 6 or an extract such as defined according to any one of claims 9 to 11.
